(19) Europäisches Patentamt
European Patent Office
Office européen des brevets

(11) Veröffentlichungsnummer: **0 529 292 A2**

# (12) EUROPÄISCHE PATENTANMELDUNG

(21) Anmeldenummer: **92112352.7**

(22) Anmeldetag: **20.07.92**

(51) Int. Cl.[5]: **C07D 403/12**, A01N 47/44, C07D 401/12, C07D 239/46

(30) Priorität: **01.08.91 DE 4125456**
**17.02.92 DE 4204715**

(43) Veröffentlichungstag der Anmeldung:
**03.03.93 Patentblatt 93/09**

(84) Benannte Vertragsstaaten:
**BE CH DE FR GB IT LI NL**

(71) Anmelder: **BAYER AG**

**W-5090 Leverkusen 1 Bayerwerk(DE)**

(72) Erfinder: **Fest, Christa, Dr.**
**Im Johannistal 20**
**W-5600 Wuppertal 1(DE)**
Erfinder: **Gesing, Ernst R., Dr.**
**Trillser Graben 4**
**W-4006 Erkrath-Hochdahl(DE)**
Erfinder: **Santel, Hans-Jochim, Dr.**
**Grünstrasse 9a**
**W-5090 Leverkusen 1(DE)**
Erfinder: **Lürssen, Klaus, Dr.**
**August-Kierspel-Strasse 156**
**W-5060 Bergisch-Gladbach 2(DE)**
Erfinder: **Schmidt, Robert R., Dr.**
**Im Waldwinkel 110**
**W-5060 Bergisch-Gladbach 2(DE)**

(54) **Substituierte Aralkylsulfonyl-aminoguanidinoazine as Herbizide.**

(57) Die Erfindung betrifft neue substituierte Aralkylsulfonylaminoguanidinoazine der allgemeinen Formel

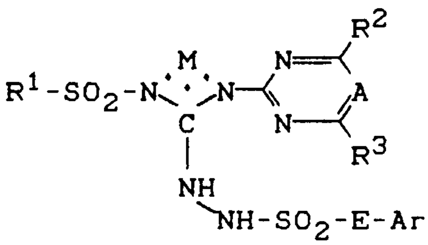

(I)

in welcher

| | |
|---|---|
| A | für Stickstoff oder eine CH-Gruppierung steht, |
| Ar | für gegebenenfalls substituiertes Aryl steht, |
| E | für Alkandiyl (Alkylen) steht, |
| M | für Wasserstoff, ein Metalläquivalent, ein Ammonium-, Alkylammonium-, Dialkylammonium- oder Trialkylammonium-Äquivalent steht, |
| $R^1$ | für jeweils gegebenenfalls substituiertes Aryl, Aralkyl oder Heteroaryl steht, sowie |
| $R^2$ und $R^3$ | gleich oder verschieden sind und für Wasserstoff, Halogen, Alkyl, Halogenalkyl, Alkoxyalkyl, Alkoxy, Halogenalkoxy, Alkoxyalkoxy, Alkylthio, Amino, Alkylamino oder Dialkylamino stehen, |

Verfahren zu ihrer Herstellung und ihre Verwendung als Herbizide.

EP 0 529 292 A2

Die Erfindung betrifft neue substituierte Aralkylsulfonylaminoguanidinoazine, Verfahren zu ihrer Herstellung und ihre Verwendung als Herbizide.

Es ist bereits bekannt, daß bestimmte Sulfonylaminoguanidinoazine, wie z.B. N'-(4,6-Dimethyl-pyrimidin-2-yl)-N''-(4-methyl-phenylsulfonylamino)-N'''-(2-chlor-phenylsulfonyl)-guanidin (vgl. EP-A 121082, EP-A 302378 und EP-A 414067), herbizide Eigenschaften aufweisen. Die Herbizidwirkung dieser bekannten Verbindungen ist jedoch nicht in allen Belangen zufriedenstellend.

Es wurden nun neue substituierte Aralkylsulfonylaminoguanidinoazine der allgemeinen Formel (I) gefunden,

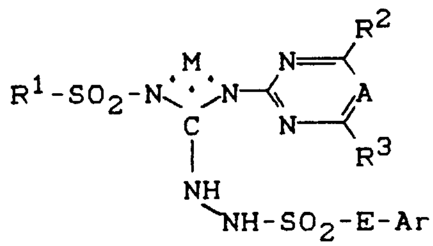

(I)

in welcher

| | |
|---|---|
| A | für Stickstoff oder eine CH-Gruppierung steht, |
| Ar | für gegebenenfalls substituiertes Aryl steht, |
| E | für Alkandiyl (Alkylen) steht, |
| M | für Wasserstoff, ein Metalläquivalent, ein Ammonium-, Alkylammonium-, Dialkylammonium- oder Trialkylammonium-Äquivalent steht, |
| $R^1$ | für jeweils gegebenenfalls substituiertes Aryl, Aralkyl oder Heteroaryl steht, sowie |
| $R^2$ und $R^3$ | gleich oder verschieden sind und für Wasserstoff, Halogen, Alkyl, Halogenalkyl, Alkoxyalkyl, Alkoxy, Halogenalkoxy, Alkoxyalkoxy, Alkylthio, Amino, Alkylamino oder Dialkylamino stehen. |

Die allgemeine Formel (I) steht - wenn M für Wasserstoff steht - für die einzelnen möglichen Tautomeren der Formeln (IA), (IB) und (IC)

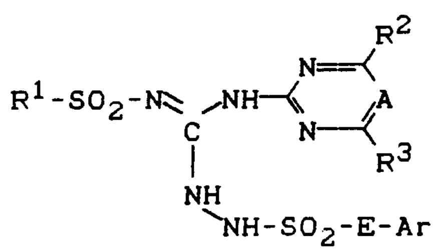

(IA)

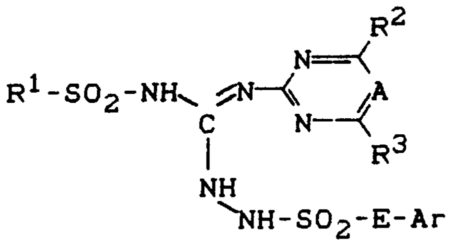

(IB)

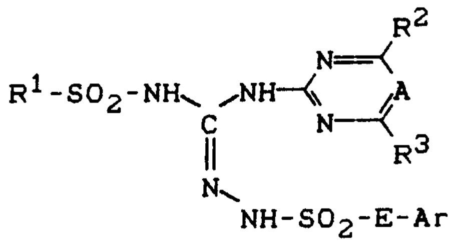

(IC)

sowie für Gemische dieser Tautomeren.

Man erhält die neuen substituierten Aralkylsulfonylaminoguanidinoazine der allgemeinen Formel (I), wenn man

(a) Sulfonylverbindungen der allgemeinen Formel (II)

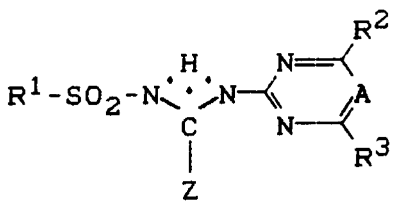

(II)

in welcher

A, $R^1$, $R^2$ und $R^3$ die oben angegebenen Bedeutungen haben und

Z für eine der nachstehend angegebenen Abgangsgruppen

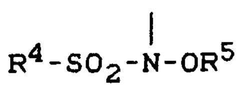

oder $-Q-R^6$ steht, worin

$R^4$ die oben für $R^1$ angegebene Bedeutung hat, aber nicht in jedem Einzelfall mit $R^1$ identisch sein muß,

$R^5$ für Alkyl, Alkenyl oder Aralkyl steht,

$R^6$ für Alkyl, Aralkyl oder Aryl steht und

Q für Sauerstoff oder Schwefel steht,

mit Sulfonsäurehydraziden der allgemeinen Formel (III)

$H_2N-NH-SO_2-E-Ar$ (III)

in welcher

Ar und E die oben angegebene Bedeutung haben,

gegebenenfalls in Gegenwart eines Verdünnungsmittels umsetzt, oder wenn man

(b) Aminoguanidinoazine der allgemeinen Formel (IV)

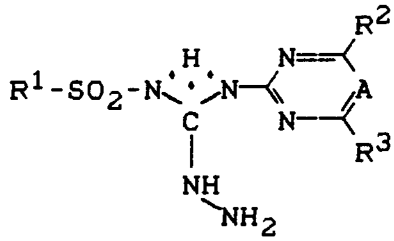

(IV)

in welcher

A, $R^1$, $R^2$ und $R^3$ die oben angegebenen Bedeutungen haben, mit Sulfonsäurehalogeniden der allgemeinen Formel (V)

$X-SO_2-E-Ar$ (V)

in welcher

Ar und E die oben angegebene Bedeutung haben und

X für Halogen steht,

gegebenenfalls in Gegenwart eines Verdünnungsmittels und gegebenenfalls in Gegenwart eines Säureakzeptors umsetzt,

und gegebenenfalls die so erhaltenen Produkte nach üblichen Methoden in Salze überführt.

Die neuen substituierten Aralkylsulfonylaminoguanidinoazine der allgemeinen Formel (I) zeichnen sich durch starke herbizide Wirksamkeit aus.

Überraschenderweise zeigen die neuen Verbindungen der allgemeinen Formel (I) erheblich bessere Wirkung als die oben erwähnten vorbekannten Sulfonylaminoguanidinoazine, welche nach Struktur und Wirkprofil vergleichbare Stoffe sind.

Gegenstand der Erfindung sind vorzugsweise Verbindungen der Formel (I), in welcher

A für Stickstoff oder eine CH-Gruppierung steht,

Ar für jeweils gegebenenfalls einfach oder zweifach substituiertes Phenyl oder Naphthyl steht, wobei die Substituenten vorzugsweise ausgewählt sind aus der Reihe Fluor, Chlor, Brom, Cyano, Nitro, Carboxy, Carbamoyl, jeweils gegebenenfalls durch Fluor und/oder Chlor einfach bis dreifach substituiertes $C_1$-$C_4$-Alkyl oder $C_1$-$C_4$-Alkoxy, jeweils gegebenenfalls durch Fluor, Chlor, Methoxy oder Ethoxy substituiertes $C_1$-$C_4$-Alkoxy-carbonyl oder $C_1$-$C_4$-Alkyl-amino-carbonyl,

E für $C_1$-$C_3$-Alkandiyl steht,

M für Wasserstoff oder ein Natrium-, Kalium- oder Calciumäquivalent, ein Ammonium-, ein $C_1$-$C_6$-Alkylammonium-, ein Di-($C_1$-$C_4$-alkyl)-ammonium- oder ein Tri-($C_1$-$C_4$-alkyl)-ammonium-äquivalent steht,

$R^1$ für den Rest

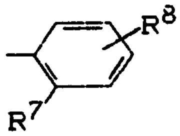

steht, worin

$R^7$ und $R^8$ gleich oder verschieden sind und für Wasserstoff, Fluor, Chlor, Brom, Iod, Cyano, Nitro, $C_1$-$C_6$-Alkyl (welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)amino-carbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)-aminosulfonyl, $C_3$-$C_6$-Cycloalkyl oder Phenyl substituiert ist), für $C_2$-$C_6$-Alkenyl (welches gegebenenfalls durch Fluor, Chlor, Brom, Cyano, $C_1$-$C_4$-Alkoxy-carbonyl, Carboxy oder Phenyl substituiert ist), für $C_2$-$C_6$-Alkinyl (welches gegebenenfalls durch Fluor, Chlor, Brom, Cyano, $C_1$-$C_4$-Alkoxy-carbonyl, Carboxy oder Phenyl substituiert ist), für $C_1$-

$C_4$-Alkoxy (welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist), für $C_1$-$C_4$-Alkylthio (welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist), für $C_3$-$C_6$-Alkenyloxy (welches gegebenenfalls durch Fluor, Chlor, Brom, Cyano oder $C_1$-$C_4$-Alkoxycarbonyl substituiert ist), für $C_2$-$C_6$-Alkenylthio (welches gegebenenfalls durch Fluor, Chlor, Brom, Cyano, Nitro, $C_1$-$C_3$-Alkylthio oder $C_1$-$C_4$-Alkoxycarbonyl substituiert ist), $C_3$-$C_6$-Alkinyloxy, $C_3$-$C_6$-Alkinylthio oder für den Rest $-S(O)_p-R^9$ stehen, wobei

p für die Zahlen 1 oder 2 steht und

$R^9$ für $C_1$-$C_4$-Alkyl (welches gegebenenfalls durch Fluor, Chlor, Brom, Cyano oder $C_1$-$C_4$-Alkoxy-carbonyl substituiert ist), $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkyl-amino, Di-($C_1$-$C_4$-alkyl)-amino oder für den Rest $-NHOR^{10}$ steht, wobei

$R^{10}$ für $C_1$-$C_6$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzhydryl oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist) steht,

$R^7$ und $R^8$ weiterhin für Phenyl oder Phenoxy, für $C_1$-$C_4$-Alkylcarbonylamino, $C_1$-$C_4$-Alkoxy-carbonylamino, $C_1$-$C_4$-Alkylamino-carbonylamino, Di-($C_1$-$C_4$-alkyl)-amino-carbonylamino, oder für den Rest $-CO-R^{11}$ stehen, wobei

$R^{11}$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$ Alkylthio, Amino, $C_1$-$C_4$-Alkyl-amino, $C_1$-$C_4$-Alkoxyamino, $C_1$-$C_4$-Alkoxy-$C_1$-$C_4$-alkyl-amino oder Di-($C_1$-$C_4$-alkyl)-amino steht (welche gegebenenfalls durch Fluor und/oder Chlor substituiert sind),

$R^7$ und $R^8$ weiterhin für $C_1$-$C_4$-Alkylsulfonyloxy, Di-($C_1$-$C_4$-alkyl)-aminosulfonylamino oder für den Rest $-CH=N-R^{12}$ stehen, wobei

$R^{12}$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/ oder Chlor substituiertes $C_1$-$C_6$-Alkoxy, $C_3$-$C_6$-Alkenoxy, $C_3$-$C_6$-Alkinoxy oder Benzyloxy,für Amino, $C_1$-$C_4$-Alkylamino, Di-($C_1$-$C_4$-alkyl)-amino, Phenylamino, $C_1$-$C_4$-Alkyl-carbonyl-amino, $C_1$-$C_4$-Alkoxy-carbonyl-amino, $C_1$-$C_4$-Alkyl-sulfonylamino oder für gegebenenfalls durch Fluor, Chlor, Brom oder Methyl substituiertes Phenylsulfonylamino steht,

weiter

$R^1$ für den Rest

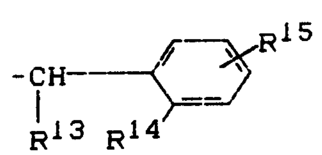

steht, worin

$R^{13}$ für Wasserstoff oder $C_1$-$C_4$-Alkyl steht,

$R^{14}$ und $R^{15}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Carboxy,

$C_1$-$C_4$-Alkoxy-carbonyl, $C_1$-$C_4$-Alkylsulfonyl oder Di-($C_1$-$C_4$-alkyl)-aminosulfonyl stehen;

weiter

$R^1$ für den Rest

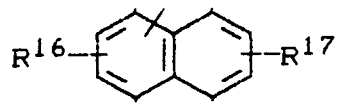

steht, worin

$R^{16}$ und $R^{17}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist) oder $C_1$-$C_4$-Alkoxy (welches gegebenenfalls durch Fluor und/oder Chlor substituiert ist), stehen;

weiter

$R^1$ für den Rest

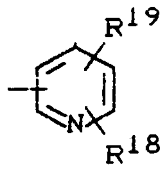

steht, worin

$R^{18}$ und $R^{19}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_2$-$C_4$-Alkenyl (welches gegebenenfalls durch Fluor und/oder Chlor substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), sowie für Di-($C_1$-$C_4$-alkyl)-aminosulfonyl, $C_1$-$C_4$-Alkoxy-carbonyl, Dimethylaminocarbonyl oder Dioxolanyl stehen;

weiter

$R^1$ für den Rest

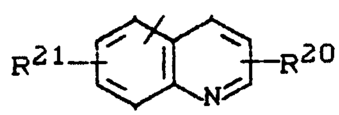

steht, worin

$R^{20}$ und $R^{21}$ gleich oder verschieden sind und für Wasserstoff, Fluor, Chlor, Brom, $C_1$-$C_4$-Alkyl (welches gegebenenfalls durch Fluor und/oder Brom substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), oder für Di-($C_1$-$C_4$-alkyl)-aminosulfonyl stehen;

weiter

$R^1$ für den Rest

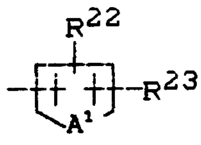

steht, worin

$R^{22}$ und $R^{23}$ gleich oder verschieden sind und für Wasserstoff, Fluor, Chlor, Brom, Cyano, Nitro, $C_1$-$C_4$-Alkyl (welches gegebenenfalls durch Fluor, Chlor, $C_1$-$C_4$-Alkoxy und/oder $C_1$-$C_4$-Halogenalkoxy substituiert ist), $C_1$-$C_4$-Alkoxy (welches gegebenenfalls durch Fluor und/oder Chlor substituiert ist), $C_1$-$C_4$-Alkylthio, $C_1$-$C_4$-Alkylsulfinyl oder $C_1$-$C_4$-Alkylsulfonyl (welches gegebenenfalls durch Fluor und/oder Chlor substituiert ist), Di-($C_1$-$C_4$-alkyl)-amino-sulfonyl oder $C_1$-$C_4$-Alkoxy-carbonyl stehen, und

$A^1$ für Sauerstoff, Schwefel oder die Gruppierung N-$Z^1$ steht, wobei

$Z^1$ für Wasserstoff, $C_1$-$C_4$-Alkyl (welches gegebenenfalls durch Fluor, Chlor, Brom oder Cyano substituiert ist), $C_3$-$C_6$-Cycloalkyl, Benzyl, Phenyl (welches gegebenenfalls durch Fluor, Chlor, Brom oder Nitro substituiert ist), $C_1$-$C_4$-Alkylcarbonyl, $C_1$-$C_4$-Alkoxy-carbonyl oder Di-($C_1$-$C_4$-alkyl)-aminocarbonyl steht;

weiter

$R^1$ für den Rest

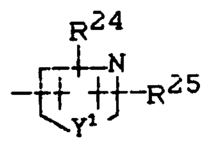

steht, worin

$R^{24}$ und $R^{25}$ gleich oder verschieden sind und für Wasserstoff, $C_1$-$C_4$-Alkyl, Halogen, $C_1$-$C_4$-Alkoxycarbonyl, $C_1$-$C_4$-Alkoxy oder $C_1$-$C_4$-Halogenalkoxy stehen,

$Y^1$ für Schwefel oder die Gruppierung N-$R^{26}$ steht, wobei

$R^{26}$ für Wasserstoff oder $C_1$-$C_4$-Alkyl steht;

weiter

$R^1$ für den Rest

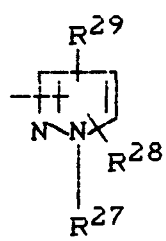

steht, worin

$R^{27}$ für Wasserstoff, $C_1$-$C_4$-Alkyl, Phenyl oder (Iso)Chinolinyl steht,

$R^{28}$ für Wasserstoff, Halogen, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Dioxolanyl oder $C_1$-$C_4$-Alkoxy-carbonyl steht und

$R^{29}$ für Wasserstoff, Halogen oder $C_1$-$C_4$-Alkyl steht; sowie

$R^2$ und $R^3$ gleich oder verschieden sind und für Wasserstoff, Fluor, Chlor, Brom, Methyl, Ethyl, Trifluormethyl, Methoxymethyl, Methoxy, Ethoxy, Difluormethoxy, Methoxyethoxy, Methylthio, Ethylthio, Amino, Methylamino, Ethylamino oder Dimethylamino stehen.

Die Erfindung betrifft insbesondere Verbindungen der Formel (I), in welcher

A für Stickstoff oder eine CH-Gruppierung steht,

Ar für gegebenenfalls einfach oder zweifach substituiertes Phenyl steht, wobei die Substituenten vorzugsweise ausgewählt sind aus der Reihe Fluor, Chlor, Brom, Carboxy, Methyl, Trifluormethyl, Methoxy, Difluormethoxy, Trifluormethoxy, Methoxycarbonyl, Ethoxycarbonyl und Cyano,

E für $-CH_2-$ oder $-CH_2CH_2-$ steht,

M für Wasserstoff steht,

$R^1$ für den Rest

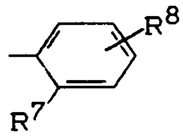

steht, worin

$R^7$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, N-Methoxy-N-methylaminosulfonyl, Phenyl, Phenoxy oder $C_1$-$C_3$-Alkoxy-carbonyl steht und

$R^8$ für Wasserstoff, Fluor oder Chlor steht;

weiter

$R^1$ für den Rest

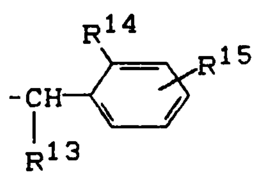

steht, worin

$R^{13}$ für Wasserstoff steht,

$R^{14}$ für Fluor, Chlor, Brom, Methyl, Methoxy, Di-fluormethoxy, Trifluormethoxy, Ethoxy, Methoxycarbonyl, Ethoxycarbonyl, Methylsulfonyl oder Dimethylaminosulfonyl steht und

$R^{15}$ für Wasserstoff, Fluor oder Chlor steht;

weiter

$R^1$ für den Rest

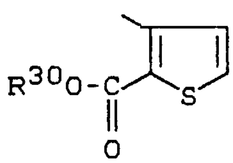

steht, worin

$R^{30}$ für Methyl oder Ethyl steht, oder

$R^1$ für den Rest

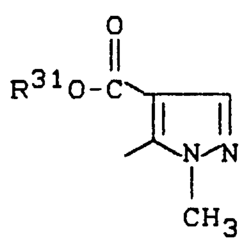

steht, worin

$R^{31}$ für Methyl oder Ethyl steht,

$R^2$ für Wasserstoff, Chlor, Methyl, Ethyl, Trifluormethyl, Methoxy oder Ethoxy steht und

$R^3$ für Methyl, Ethyl, Methoxy oder Ethoxy steht.

Verwendet man beispielsweise N'-(4-Methoxy-6-methyl-s-triazin-2-yl)-N''-(2-fluor-phenylsulfonyl)-O-methylisoharnstoff und Phenylmethansulfonsäurehydrazid als Ausgangsstoffe, so kann der Reaktionsablauf beim erfindungsgemäßen Verfahren (a) durch das folgende Formelschema skizziert werden;

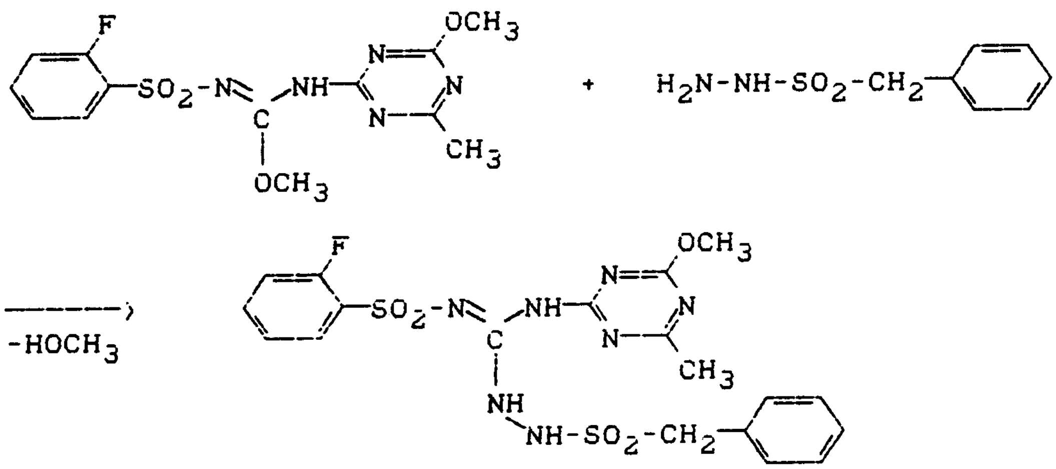


Verwendet man beispielsweise N'-(4-Chlor-6-methoxy-pyrimidin-2-yl)-N''-(2-trifluormethyl-phenylsulfonyl)-N'''-amino-guanidin und 4-Methoxycarbonyl-phenylmethansulfon-säurechlorid als Ausgangsstoffe, so kann der Reaktionsablauf beim erfindungsgemäßen Verfahren (b) durch das folgende Formelschema skizziert werden:

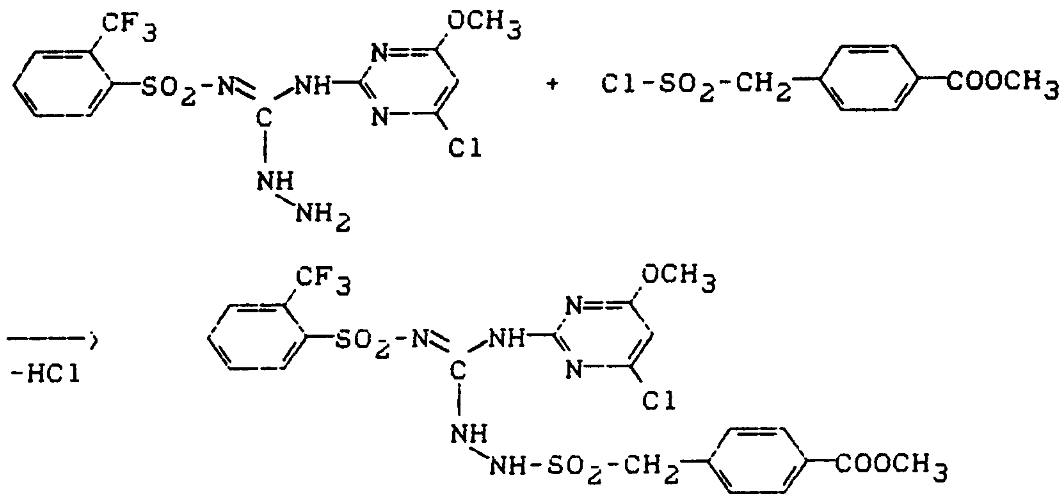


Die beim erfindungsgemäßen Verfahren (a) zur Herstellung von Verbindungen der Formel (I) als Ausgangsstoffe zu verwendenden Sulfonylverbindungen sind durch die Formel (II) allgemein definiert.

In Formel (II) haben A, $R^1$, $R^2$ und $R^3$ vorzugsweise bzw, insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, $R^1$, $R^2$ und $R^3$ angegeben wurden;

Z steht vorzugsweise für eine der nachstehend angegebenen Abgangsgruppen

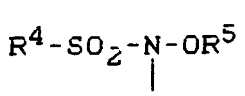

oder $-Q-R^6$ , worin

$R^4$ die oben für $R^1$ vorzugsweise angegebene Bedeutung hat, aber nicht in jedem Einzelfall mit $R^1$ identisch sein muß,

$R^5$ für $C_1$-$C_4$-Alkyl, $C_3$-$C_4$-Alkenyl oder Benzyl steht,

$R^6$ für $C_1$-$C_4$-Alkyl, Benzyl oder Phenyl steht und

Q für Sauerstoff oder Schwefel steht.

Die Ausgangsstoffe der Formel (II) sind bekannt und/oder können nach an sich bekannten Verfahren hergestellt werden (vgl. EP-A 121 082, EP-A 172 957, EP-A 173 321, EP-A 173 956, EP-A 224 078, EP-A 5 986, EP-A 24 215, EP-A 302 378).

Die beim erfindungsgemäßen Verfahren weiter als Ausgangsstoffe zu verwendenden Sulfonsäurehydrazide sind durch die Formel (III) allgemein definiert. In Formel (III) haben Ar und E vorzugsweise bzw. insbesondere diejenige Bedeutung, die bereits oben im Rahmen der Beschreibung der erfindungsgemäßen Verbindungen der Formel (I) als bevorzugt bzw. als insbesondere bevorzugt für Ar und E angegeben wurde.

Die Ausgangsstoffe der Formel (III) sind bekannt und/oder können nach an sich bekannten Verfahren hergestellt werden (vgl. DE-OS 2 264 363, US-P 4 218 465, J. Pharm. Sci. 68 (1979), 930-931).

Das erfindungsgemäße Verfahren (a) zur Herstellung der neuen Verbindungen der Formel (I) wird vorzugsweise unter Verwendung von Verdünnungsmitteln durchgeführt. Als Verdünnungsmittel kommen dabei vorzugsweise Wasser und/oder polare organische Lösungsmittel, wie Methanol, Ethanol, Isopropanol, Butanol, Isobutanol, sec-Butanol, tert-Butanol, Glycoldimethylether, Diglycoldimethylether, Tetrahydrofuran, Dioxan, Essigsäuremethylester, Essigsäureethylester, Acetonitril, Propionitril, Dimethylformamid, Dimethylacetamid, N-Methylpyrrolidon, Dimethylsulfoxid und Tetramethylensulfon, in Betracht.

Die Reaktionstemperaturen können bei dem erfindungsgemäßen Verfahren (a) in einem größeren Bereich variiert werden. Im allgemeinen arbeitet man bei Temperaturen zwischen 0 °C und 150 °C, vorzugsweise bei Temperaturen zwischen 10 °C und 100 °C.

Zur Durchführung des erfindungsgemäßen Verfahrens (a) setzt man je Mol Sulfonylverbindung der Formel (II) im allgemeinen zwischen 1 und 5 Mol, vorzugsweise zwischen 1 und 3 Mol, Sulfonsäurehydrazid der Formel (III) ein.

Im allgemeinen werden die Reaktionskomponenten bei Raumtemperatur oder unter Eiskühlung zusammen gegeben und das Reaktionsgemisch wird, gegebenenfalls bei erhöhter Temperatur, bis zum Ende der Umsetzung gerührt. Die Produkte der Formel (I) fallen im allgemeinen nach dem Abkühlen kristallin an und können durch Absaugen isoliert werden.

Die beim erfindungsgemäßen Verfahren (b) zur Herstellung von Verbindungen der Formel (I) als Ausgangsstoffe zu verwendenden Aminoguanidinoazine sind durch die Formel (IV) allgemein definiert.

In Formel (IV) haben A, $R^1$, $R^2$ und $R^3$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für A, $R^1$, $R^2$ und $R^3$ angegeben wurden.

Die Ausgangsstoffe der Formel (IV) sind bekannt und/oder können nach an sich bekannten Verfahren hergestellt werden (vgl. EP-A 224 078, US-P 4 725 303, EP-A 343 462, EP-A 414 067).

Die beim erfindungsgemäßen Verfahren (b) weiter als Ausgangsstoffe zu verwendenden Sulfonsäurehalogenide sind durch die Formel (V) allgemein definiert.

In Formel (V) haben Ar und E vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für Ar und E angegeben wurde und X steht vorzugsweise für Fluor, Chlor oder Brom, insbesondere für Chlor.

Die Ausgangsstoffe der Formel (V) sind bekannt und/oder können nach an sich bekannten Verfahren hergestellt werden (vgl. EP-A 173 321, EP-A 271 780 und EP-A 447 645).

Das erfindungsgemäße Verfahren (b) zur Herstellung der neuen Verbindungen der Formel (I) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren (b) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2, 2]-octan (DABCO).

Die Reaktionstemperaturen können bei dem erfindungsgemäßen Verfahren (b) in einem größeren Bereich variiert werden. Im allgemeinen arbeitet man bei Temperaturen zwischen -100°C und +100°C, vorzugsweise bei Temperaturen zwischen -50°C und +50°C.

Das erfindungsgemäße Verfahren (b) wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel, gegebenenfalls in Gegenwart eines Säureakzeptors, durchgeführt, und das Reaktionsgemisch wird mehrere Stunden bei der jeweils erforderlichen Temperatur gerührt. Die Aufarbeitung erfolgt bei dem erfindungsgemäßen Verfahren (b) jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen eignen sich insbesondere zur selektiven Bekämpfung von monokotylen und dikotylen Unkräutern in monokotylen Kulturen, sowohl im Vorauflauf- als auch im Nachauflauf-Verfahren.

In gewissem Umfang zeigen sie auch Wirkung gegen Pyricularia oryzae am Reis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage:
z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Ouizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

Die oben aufgeführten allgemeinen oder in Verzugsbereichen aufgeführten Restedefinitionen können untereinander, also auch zwischen den jeweiligen Vorzugsbereichen beliebig kombiniert werden.

Herstellungsbeispiele:

Beispiel 1

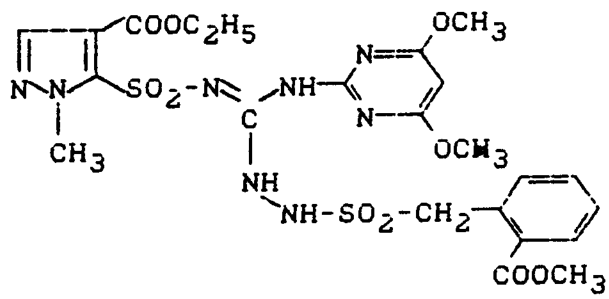

(Verfahren (b))

7,5 g (0,03 Mol) 2-Methoxycarbonyl-phenylmethansulfon-säurechlorid werden unter Rühren zu einer auf -5°C abgekühlten Mischung aus 12,8 g (0,03 Mol) N'-(4,6-Dimethoxy-pyrimidin-2-yl)-N''-amino-N'''-(1-methyl-4-ethoxycarbonyl-pyrazol-5-yl-sulfonyl)-guanidin und 100 ml Pyridin gegeben. Das Reaktionsgemisch wird noch eine Stunde bei -5°C und dann weitere 15 Stunden bei 20°C gerührt. Dann wird im Wasserstrahlvakuum eingeengt, der Rückstand in Methylenchlorid aufgenommen, zweimal mit Wasser gewaschen, mit Natriumsulfat getrocknet und filtriert. Das Filtrat wird eingeengt und der Rückstand aus Isopropanol umkristallisiert.

Man erhält 5,6 g (28% der Theorie) N'-(4,6-Dimethoxy-pyrimidin-2-yl)-N''-(2-methoxycarbonyl-phenylmethylsulfonylamino)-N'''-(1-methyl-4-ethoxycarbonyl-pyrazol-5-yl-sulfonyl)-guanidin vom Schmelzpunkt 128°C.

Analog zu Beispiel 1 und entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

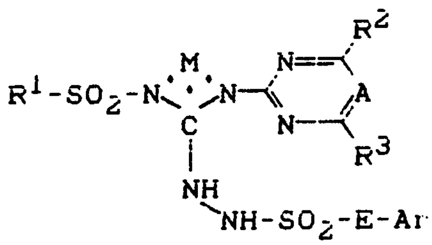

(I)

**Tabelle 1:** Beispiele für die Verbindungen der Formel (I)

| Bsp.-Nr. | A | Ar | E | M | $R^1$ | $R^2$ | $R^3$ | Schmelzpunkt (°C) |
|---|---|---|---|---|---|---|---|---|
| 2 | CH | 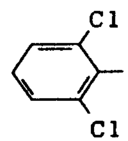 | $CH_2$ | H | 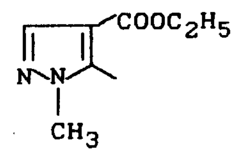 | $OCH_3$ | $OCH_3$ | 187 |
| 3 | CH | 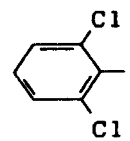 | $CH_2$ | H | 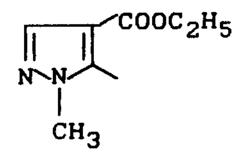 | $CH_3$ | $CH_3$ | 187 |
| 4 | CH | 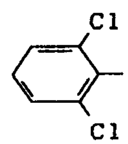 | $CH_2$ | H | 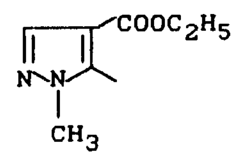 | $CH_3$ | $OCH_3$ | 224 |
| 5 | CH | 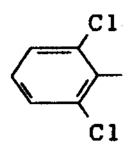 | $CH_2$ | H | 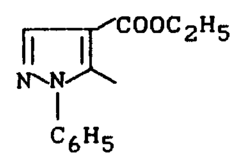 | $CH_3$ | $CH_3$ | 207 |
| 6 | CH | 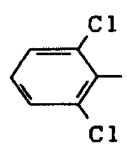 | $CH_2$ | H | 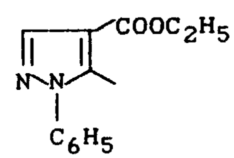 | $OCH_3$ | $OCH_3$ | 193 |

<u>Tabelle 1</u> - Fortsetzung

| Bsp.-Nr. | A | Ar | E | M | $R^1$ | $R^2$ | $R^3$ | Schmelzpunkt (°C) |
|---|---|---|---|---|---|---|---|---|
| 7 | CH | 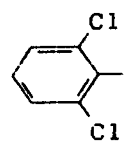 | $CH_2$ | H | 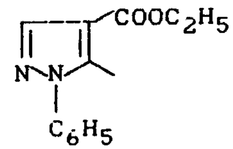 | $CH_3$ | $OCH_3$ | 192 |
| 8 | CH | 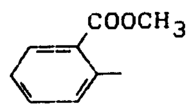 | $CH_2$ | H | 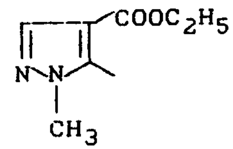 | $CH_3$ | $OCH_3$ | 140 |
| 9 | CH | 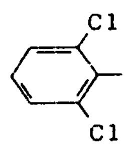 | $CH_2$ | H | 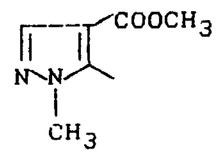 | H | $CH_3$ | 134 |
| 10 | CH | 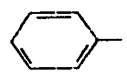 | $CH_2$ | H | 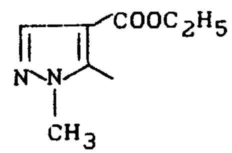 | $OCH_3$ | $OCH_3$ | 134 |
| 11 | CH | 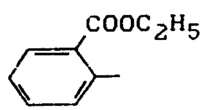 | $CH_2$ | H | 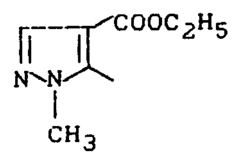 | $OCH_3$ | $OCH_3$ | 131 |

<u>Tabelle 1</u> - Fortsetzung

| Bsp.-Nr. | A | Ar | E | M | $R^1$ | $R^2$ | $R^3$ | Schmelzpunkt (°C) |
|---|---|---|---|---|---|---|---|---|
| 12 | CH | 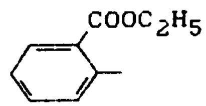 | $CH_2$ | H | 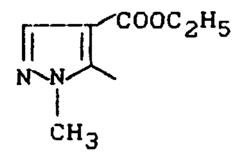 | $CH_3$ | $OCH_3$ | 153 |
| 13 | CH | 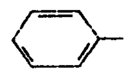 | $CH_2$ | H | 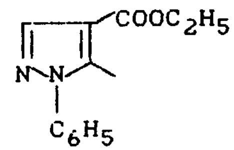 | $OCH_3$ | $OCH_3$ | 144 |
| 14 | CH | 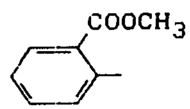 | $CH_2$ | H | 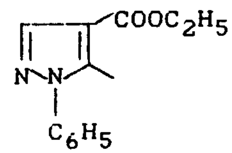 | $CH_3$ | $OCH_3$ | 173 |
| 15 | CH | 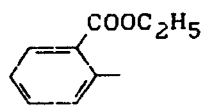 | $CH_2$ | H | 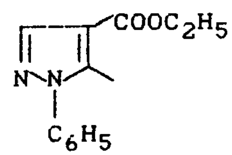 | $CH_3$ | $OCH_3$ | 195 |
| 16 | CH | 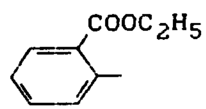 | $CH_2$ | H | 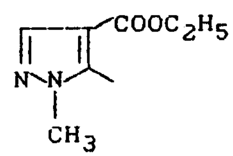 | $OCH_3$ | $OCH_3$ | 118 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 17 | CH | 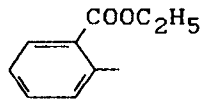 | $CH_2$ | H | 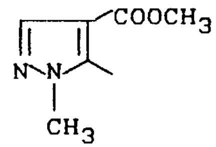 | $CH_3$ | $OCH_3$ | 163 |
| 18 | CH | 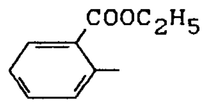 | $CH_2$ | H | 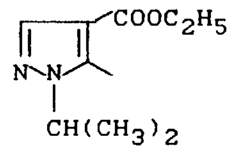 | $OCH_3$ | $OCH_3$ | 125 |
| 19 | CH | 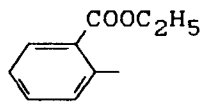 | $CH_2$ | H | 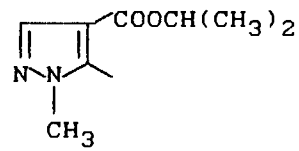 | $OCH_3$ | $OCH_3$ | 151 |
| 20 | CH | 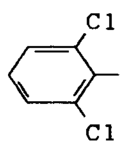 | $CH_2$ | H | 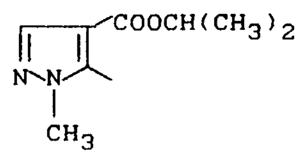 | $OCH_3$ | $OCH_3$ | 185 |
| 21 | CH | 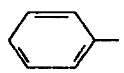 | $CH_2$ | H | 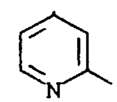 | $CH_3$ | $OCH_3$ | 88 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 22 | CH | 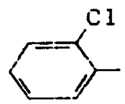 | $CH_2$ | H | 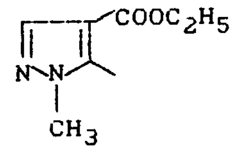 | $OCH_3$ | $OCH_3$ | 158 |
| 23 | CH | 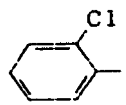 | $CH_2$ | H | 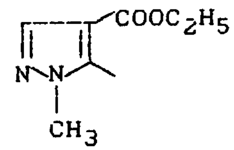 | $CH_3$ | $OCH_3$ | 162 |
| 24 | CH | 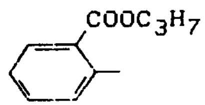 | $CH_2$ | H | 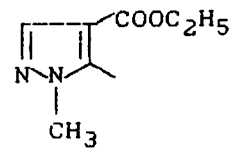 | $CH_3$ | $OCH_3$ | 147 |
| 25 | CH | 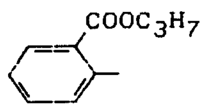 | $CH_2$ | H | 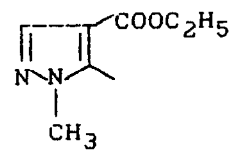 | $OCH_3$ | $OCH_3$ | 121 |
| 26 | CH | 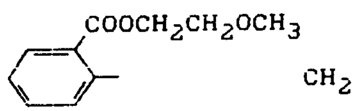 | $CH_2$ | H | 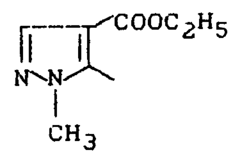 | $CH_3$ | $OCH_3$ | 166 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 27 | CH | 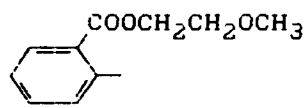 | $CH_2$ | H | 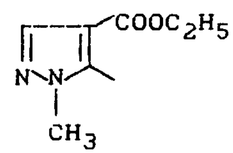 | $OCH_3$ | $OCH_3$ | 127 |
| 28 | CH | 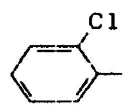 | $CH_2$ | H | 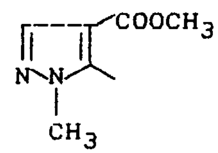 | $OCH_3$ | $OCH_3$ | 143 |
| 29 | CH | 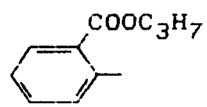 | $CH_2$ | H | 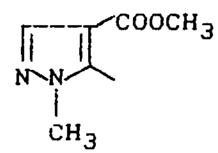 | $OCH_3$ | $OCH_3$ | 144 |
| 30 | CH | 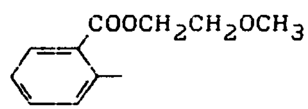 | $CH_2$ | H | 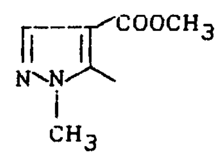 | $OCH_3$ | $OCH_3$ | 165 |
| 31 | CH | 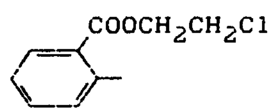 | $CH_2$ | H | 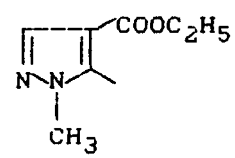 | $CH_3$ | $OCH_3$ | 158 |

<u>Tabelle 1</u> - Fortsetzung

| Bsp.-Nr. | A | Ar | E | M | $R^1$ | $R^2$ | $R^3$ | Schmelzpunkt (°C) |
|---|---|---|---|---|---|---|---|---|
| 32 | CH | 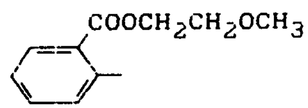 | $CH_2$ | H | 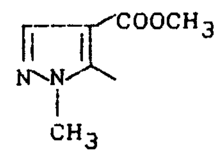 | $CH_3$ | $OCH_3$ | 159 |
| 33 | CH | 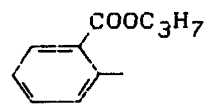 | $CH_2$ | H | 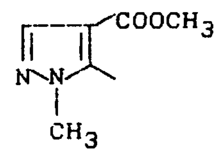 | $CH_3$ | $OCH_3$ | 168 |
| 34 | CH | 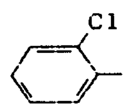 | $CH_2$ | H | 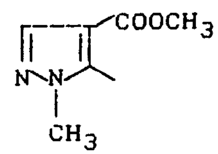 | $CH_3$ | $OCH_3$ | 150 |
| 35 | CH | 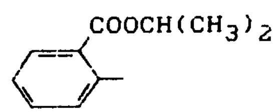 | $CH_2$ | H | 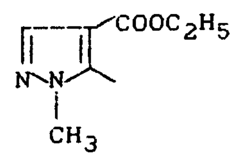 | $CH_3$ | $OCH_3$ | 169 |
| 36 | CH | 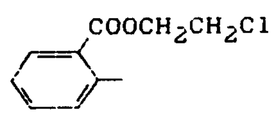 | $CH_2$ | H | 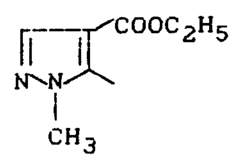 | $OCH_3$ | $OCH_3$ | 150 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 37 | CH | 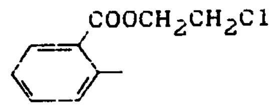 | $CH_2$ | H | 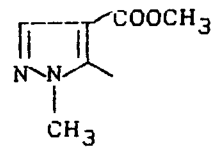 | $CH_3$ | $OCH_3$ | 165 |
| 38 | CH | 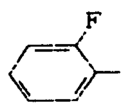 | $CH_2$ | H | 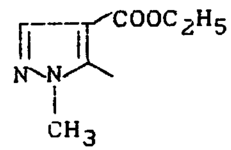 | $CH_3$ | $OCH_3$ | 154 |
| 39 | CH | 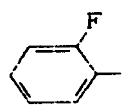 | $CH_2$ | H | 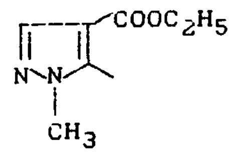 | $OCH_3$ | $OCH_3$ | 149 |
| 40 | CH | 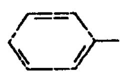 | $CH_2$ | H | 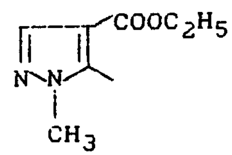 | $CH_3$ | $OCH_3$ | 138 |
| 41 | CH | 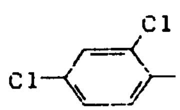 | $CH_2$ | H | 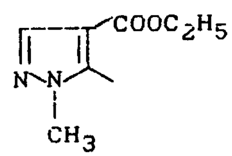 | $CH_3$ | $OCH_3$ | 166 |

<u>Tabelle 1</u> - Fortsetzung

| Bsp.- Nr. | A | Ar | E | M | $R^1$ | $R^2$ | $R^3$ | Schmelzpunkt (°C) |
|---|---|---|---|---|---|---|---|---|
| 42 | CH | 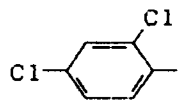 | $CH_2$ | H | 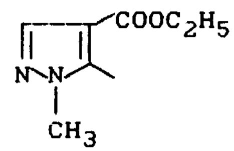 | $OCH_3$ | $OCH_3$ | 177 |
| 43 | CH | 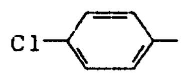 | $CH_2$ | H | 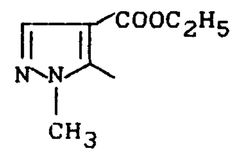 | $CH_3$ | $OCH_3$ | 125 |
| 44 | CH | 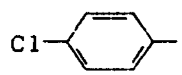 | $CH_2$ | H | 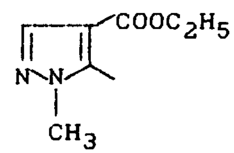 | $OCH_3$ | $OCH_3$ | 104 |
| 45 | CH | 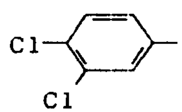 | $CH_2$ | H | 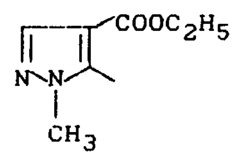 | $CH_3$ | $OCH_3$ | 124 |
| 46 | CH | 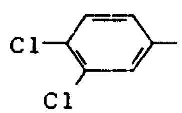 | $CH_2$ | H | 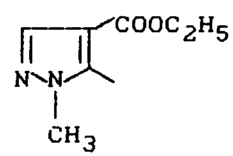 | $OCH_3$ | $OCH_3$ | 84 |

<u>Tabelle 1</u> - Fortsetzung

| Bsp.-Nr. | A | Ar | E | M | $R^1$ | $R^2$ | $R^3$ | Schmelzpunkt (°C) |
|---|---|---|---|---|---|---|---|---|
| 47 | CH | 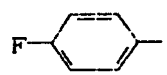 | $CH_2$ | H | 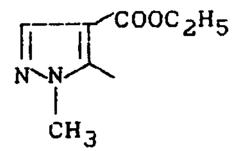 | $CH_3$ | $OCH_3$ | 183 |
| 48 | CH | 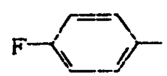 | $CH_2$ | H | 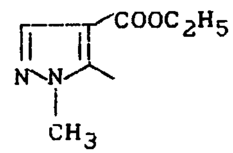 | $OCH_3$ | $OCH_3$ | 154 |
| 49 | CH | 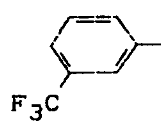 | $CH_2$ | H | 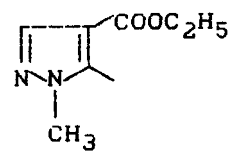 | $CH_3$ | $OCH_3$ | 162 |
| 50 | CH | 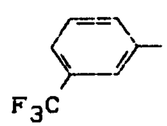 | $CH_2$ | H | 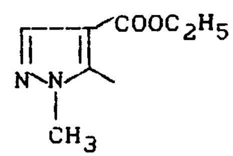 | $OCH_3$ | $OCH_3$ | 152 |
| 51 | CH | 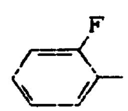 | $CH_2$ | H | 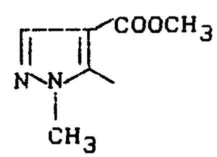 | $CH_3$ | $OCH_3$ | 144 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 52 | CH | 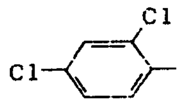 | $CH_2$ | H | 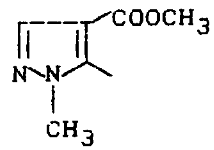 | $CH_3$ | $OCH_3$ | 138 |
| 53 | CH | 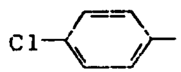 | $CH_2$ | H | 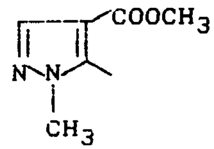 | $CH_3$ | $OCH_3$ | 134 |
| 54 | CH | 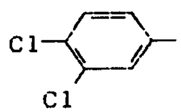 | $CH_2$ | H | 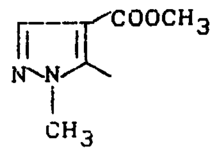 | $CH_3$ | $OCH_3$ | 142 |
| 55 | CH | 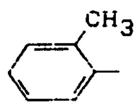 | $CH_2$ | H | 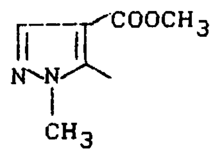 | $CH_3$ | $OCH_3$ | 84 |
| 56 | CH | 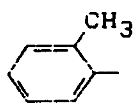 | $CH_2$ | H | 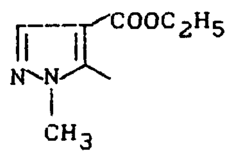 | $CH_3$ | $OCH_3$ | 91 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 57 | CH | 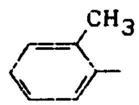 | $CH_2$ | H | 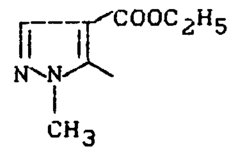 | $OCH_3$ | $OCH_3$ | 128 |
| 58 | CH | 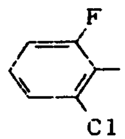 | $CH_2$ | H | 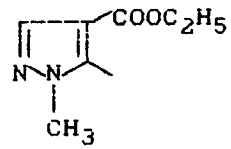 | $CH_3$ | $OCH_3$ | 211 |
| 59 | CH | 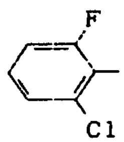 | $CH_2$ | H | 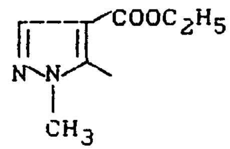 | $OCH_3$ | $OCH_3$ | 170 |
| 60 | CH | 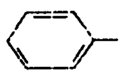 | $CH_2$ | H | 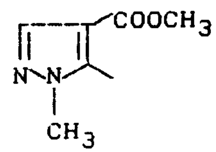 | $OCH_3$ | $OCH_3$ | 145 |
| 61 | CH | 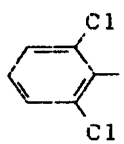 | $CH_2$ | H | 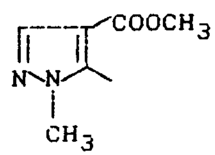 | $OCH_3$ | $OCH_3$ | 202 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 62 | CH | 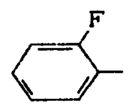 | $CH_2$ | H | 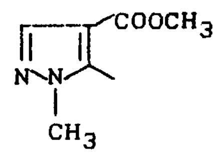 | $OCH_3$ | $OCH_3$ | 135 |
| 63 | CH | 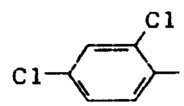 | $CH_2$ | H | 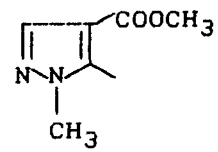 | $OCH_3$ | $OCH_3$ | 132 |
| 64 | CH | 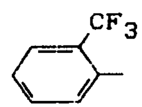 | $CH_2$ | H | 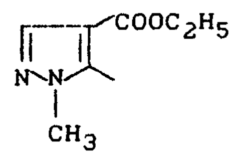 | $CH_3$ | $OCH_3$ | 173 |
| 65 | CH | 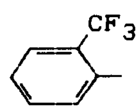 | $CH_2$ | H | 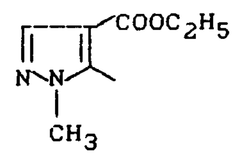 | $OCH_3$ | $OCH_3$ | 142 |
| 66 | CH | 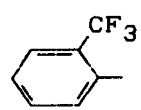 | $CH_2$ | H | 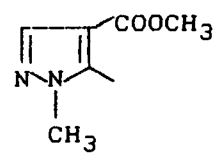 | $OCH_3$ | $OCH_3$ | 181 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 67 | CH | 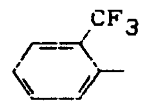 | $CH_2$ | H | 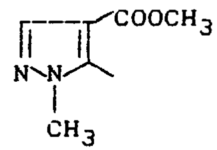 | $CH_3$ | $OCH_3$ | 184 |
| 68 | CH | 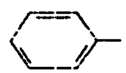 | $CH_2$ | H | 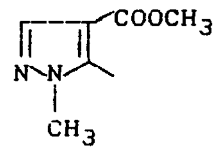 | $CH_3$ | $OCH_3$ | 160 |
| 69 | CH | 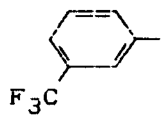 | $CH_2$ | H | 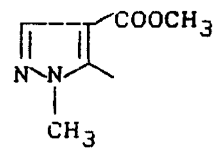 | $OCH_3$ | $OCH_3$ | 111 |
| 70 | CH | 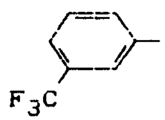 | $CH_2$ | H | 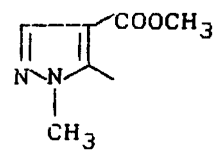 | $CH_3$ | $OCH_3$ | 163 |
| 71 | CH | 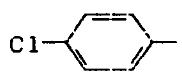 | $CH_2$ | H | 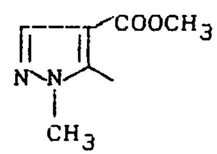 | $OCH_3$ | $OCH_3$ | 173 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 72 | CH | 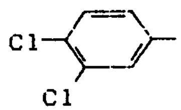 | $CH_2$ | H | 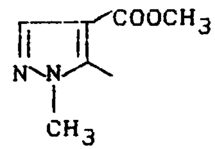 | $OCH_3$ | $OCH_3$ | 192 |
| 73 | CH | 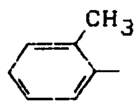 | $CH_2$ | H | 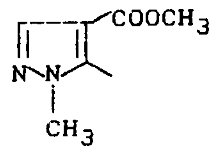 | $OCH_3$ | $OCH_3$ | 145 |
| 74 | CH | 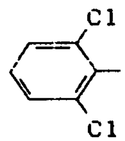 | $CH_2$ | H | 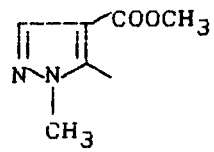 | $CH_3$ | $OCH_3$ | 210 |
| 75 | CH | 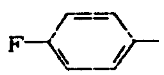 | $CH_2$ | H | 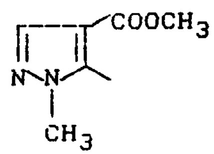 | $OCH_3$ | $OCH_3$ | 174 |
| 76 | CH | 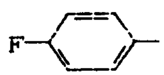 | $CH_2$ | H | 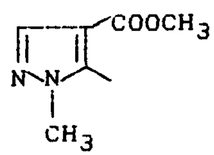 | $CH_3$ | $OCH_3$ | 177 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 77 | CH | 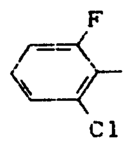 | $CH_2$ | H | 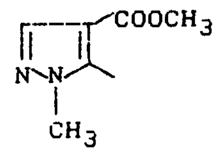 | $OCH_3$ | $OCH_3$ | |
| 78 | CH | 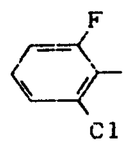 | $CH_2$ | H | 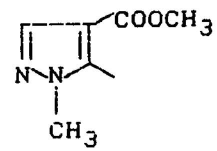 | $CH_3$ | $OCH_3$ | 170 |
| 79 | CH | 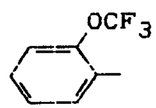 | $CH_2$ | H | 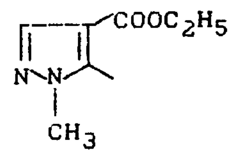 | $CH_3$ | $OCH_3$ | 140 |
| 80 | CH | 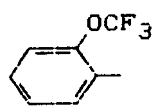 | $CH_2$ | H | 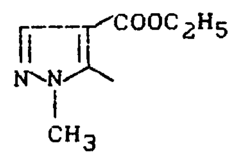 | $OCH_3$ | $OCH_3$ | 123 |
| 81 | CH | 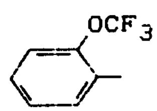 | $CH_2$ | H | 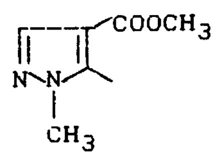 | $CH_3$ | $OCH_3$ | 191 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 82 | N | 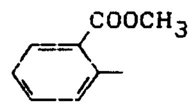 | $CH_2$ | H | 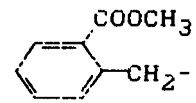 | $CH_3$ | $OCH_3$ | |
| 83 | CH | 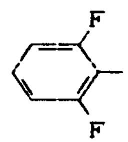 | $CH_2$ | H | 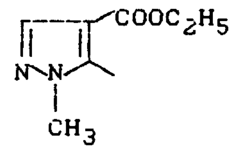 | $CH_3$ | $OCH_3$ | 185 |
| 84 | CH | 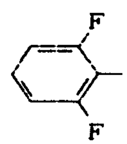 | $CH_2$ | H | 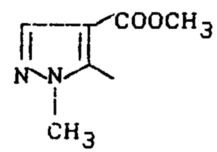 | $CH_3$ | $OCH_3$ | 165 |
| 85 | CH | 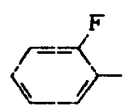 | $CH_2$ | H | 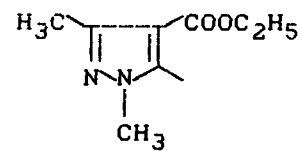 | $OCH_3$ | $OCH_3$ | 139 |
| 86 | CH | 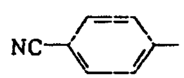 | $CH_2$ | H | 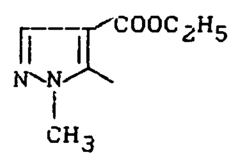 | $CH_3$ | $OCH_3$ | 151 |

<u>Tabelle 1</u> - Fortsetzung

| Bsp.-Nr. | A | Ar | E | M | $R^1$ | $R^2$ | $R^3$ | Schmelzpunkt (°C) |
|---|---|---|---|---|---|---|---|---|
| 87 | CH | 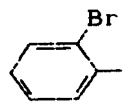 | $CH_2$ | H | 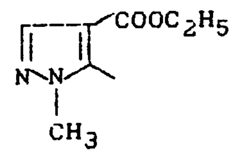 | $OCH_3$ | $OCH_3$ | 130 |
| 88 | CH | 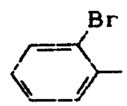 | $CH_2$ | H | 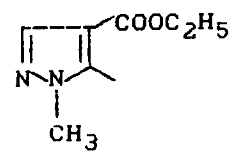 | $CH_3$ | $OCH_3$ | 146 |
| 89 | CH | 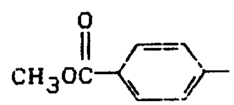 | $CH_2$ | H | 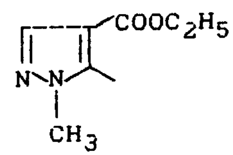 | $CH_3$ | $OCH_3$ | 142 |
| 90 | CH | 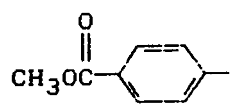 | $CH_2$ | H | 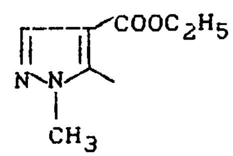 | $OCH_3$ | $OCH_3$ | 167 |
| 91 | CH | 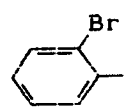 | $CH_2$ | H | 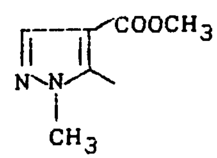 | $CH_3$ | $OCH_3$ | 135 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 92 | CH | 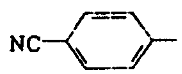 | $CH_2$ | H | 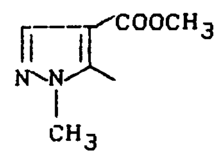 | $OCH_3$ | $OCH_3$ | 172 (Zers.) |
| 93 | CH | 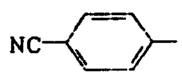 | $CH_2$ | H | 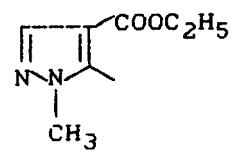 | $OCH_3$ | $OCH_3$ | 164 (Zers.) |
| 94 | CH | 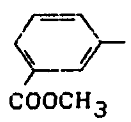 | $CH_2$ | H | 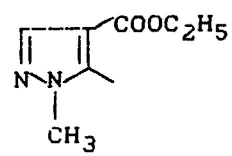 | $CH_3$ | $OCH_3$ | 150 |
| 95 | CH | 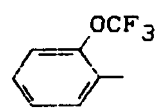 | $CH_2$ | H | 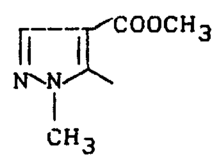 | $OCH_3$ | $OCH_3$ | 167 |
| 96 | CH | 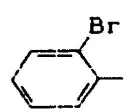 | $CH_2$ | H | 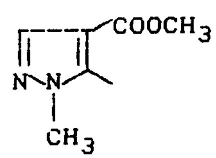 | $OCH_3$ | $OCH_3$ | 123 |

<u>Tabelle 1</u> - Fortsetzung

| Bsp.-Nr. | A | Ar | E | M | $R^1$ | $R^2$ | $R^3$ | Schmelz-punkt (°C) |
|---|---|---|---|---|---|---|---|---|
| 97 | CH | 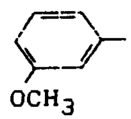 | $CH_2$ | H | 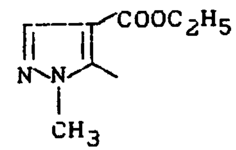 | $CH_3$ | $OCH_3$ | 73 (Zers.) |
| 98 | CH | 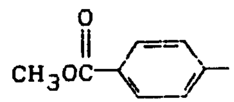 | $CH_2$ | H | 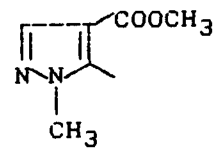 | $OCH_3$ | $OCH_3$ | 173 (Zers.) |
| 99 | CH | 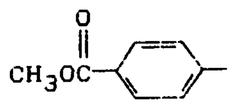 | $CH_2$ | H | 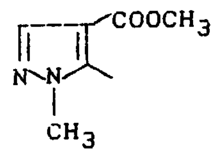 | $OCH_3$ | $CH_3$ | 171 (Zers.) |
| 100 | CH | 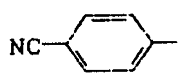 | $CH_2$ | H | 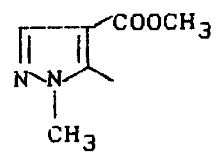 | $CH_3$ | $OCH_3$ | 153 (Zers.) |
| 101 | CH | 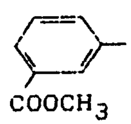 | $CH_2$ | H | 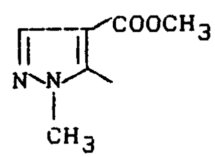 | $OCH_3$ | $OCH_3$ | 155 (Zers.) |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 102 | CH | 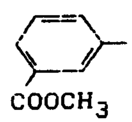 | $CH_2$ | H | 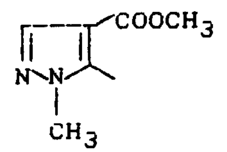 | $CH_3$ | $OCH_3$ | 151 (Zers.) |
| 103 | CH | 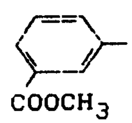 | $CH_2$ | H | 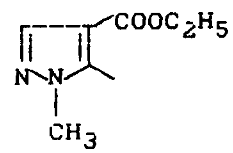 | $OCH_3$ | $OCH_3$ | 154 |
| 104 | CH | 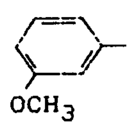 | $CH_2$ | H | 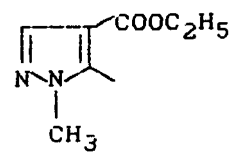 | $OCH_3$ | $OCH_3$ | 156 |
| 105 | CH | 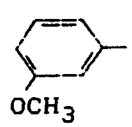 | $CH_2$ | H | 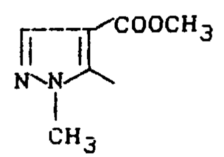 | $OCH_3$ | $OCH_3$ | 115 |
| 106 | CH | 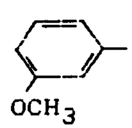 | $CH_2$ | H | 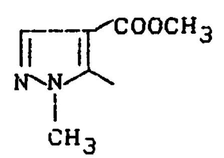 | $CH_3$ | $OCH_3$ | 153 |

<u>Tabelle 1</u> - Fortsetzung

| Bsp.-Nr. | A | Ar | E | M | $R^1$ | $R^2$ | $R^3$ | Schmelzpunkt (°C) |
|---|---|---|---|---|---|---|---|---|
| 107 | CH | 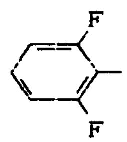 | $CH_2$ | H | 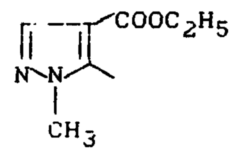 | $OCH_3$ | $OCH_3$ | 167 |
| 108 | CH | 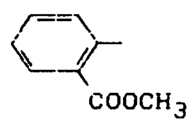 | $CH_2$ | H | 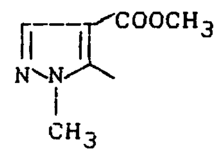 | $CH_3$ | $OCH_3$ | 180 |
| 109 | CH | 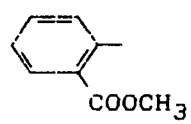 | $CH_2$ | H | 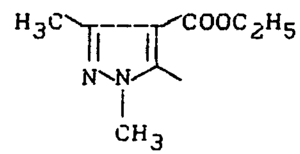 | $OCH_3$ | $OCH_3$ | |
| 110 | CH | 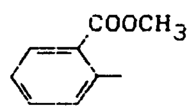 | $CH_2CH_2$ | H | 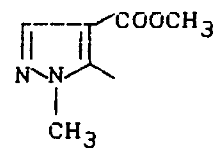 | $OCH_3$ | $CF_3$ | |
| 111 | CH | 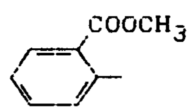 | $CH_2$ | H | 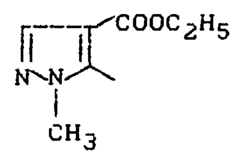 | $OCH_3$ | $CF_3$ | |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 112 | CH | 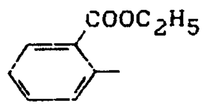 | $CH_2$ | H | 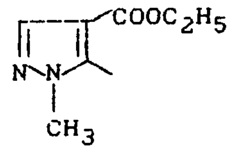 | $OCH_3$ | $CF_3$ | |
| 113 | CH | 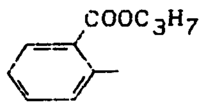 | $CH_2$ | H | 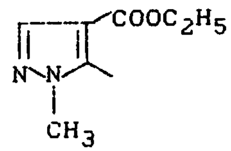 | $OCH_3$ | $CF_3$ | |
| 114 | CH | 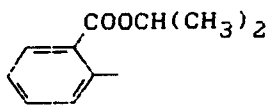 | $CH_2$ | H | 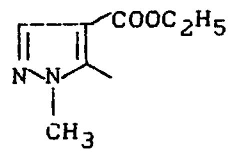 | $OCH_3$ | $CF_3$ | |
| 115 | CH | 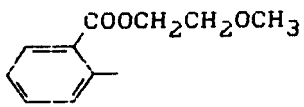 | $CH_2$ | H | 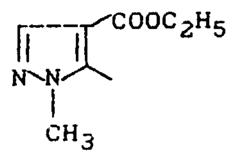 | $OCH_3$ | $CF_3$ | |
| 116 | CH | 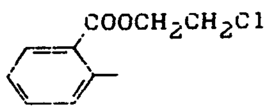 | $CH_2$ | H | 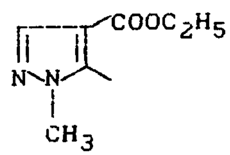 | $OCH_3$ | $CF_3$ | |

<u>Tabelle 1</u> - Fortsetzung

| Bsp.-Nr. | A | Ar | E | M | $R^1$ | $R^2$ | $R^3$ | Schmelzpunkt (°C) |
| --- | --- | --- | --- | --- | --- | --- | --- | --- |
| 117 | CH | 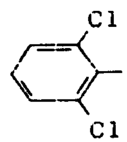 | $CH_2$ | H | 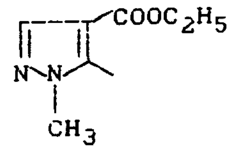 | $OCH_3$ | $CF_3$ | |
| 118 | CH | 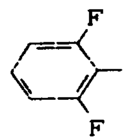 | $CH_2$ | H | 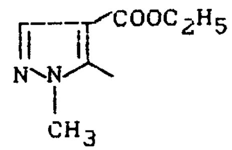 | $OCH_3$ | $CF_3$ | |
| 119 | CH | 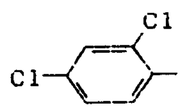 | $CH_2$ | H | 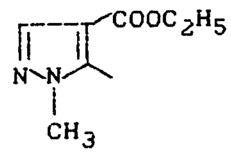 | $OCH_3$ | $CF_3$ | |
| 120 | CH | 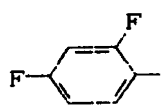 | $CH_2$ | H | 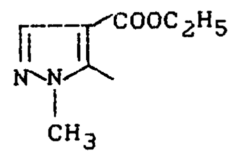 | $OCH_3$ | $CF_3$ | |
| 121 | CH | 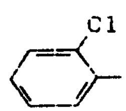 | $CH_2CH_2$ | H | 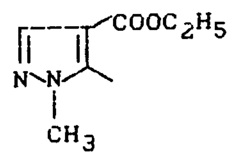 | $OCH_3$ | $CF_3$ | |

<u>Tabelle 1</u> - Fortsetzung

| Bsp.-Nr. | A | Ar | E | M | $R^1$ | $R^2$ | $R^3$ | Schmelzpunkt (°C) |
|---|---|---|---|---|---|---|---|---|
| 122 | CH | 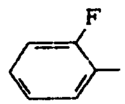 | $CH_2$ | H | 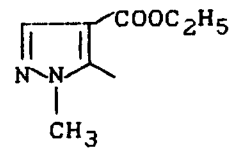 | $OCH_3$ | $CF_3$ | |
| 123 | CH | 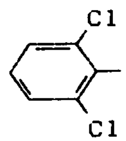 | $CH_2$ | H | 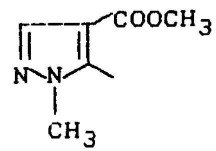 | $OCH_3$ | $CF_3$ | |
| 124 | CH | 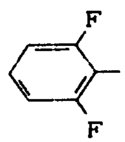 | $CH_2$ | H | 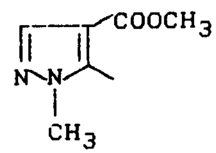 | $OCH_3$ | $CF_3$ | |
| 125 | CH | 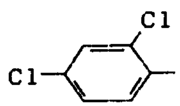 | $CH_2$ | H | 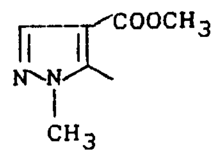 | $OCH_3$ | $CF_3$ | |
| 126 | CH | 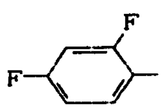 | $CH_2$ | H | 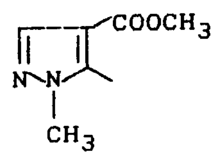 | $OCH_3$ | $CF_3$ | |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 127 | CH | 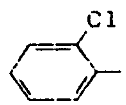 | $CH_2CH_2$ | H | 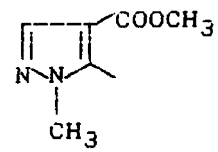 | $OCH_3$ | $CF_3$ | |
| 128 | CH | 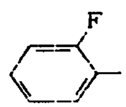 | $CH_2$ | H | 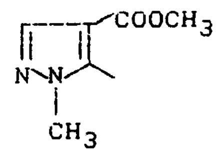 | $OCH_3$ | $CF_3$ | |
| 129 | CH | 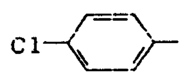 | $CH_2$ | H | 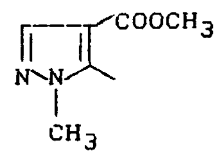 | $OCH_3$ | $CF_3$ | |
| 130 | CH | 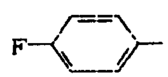 | $CH_2$ | H | 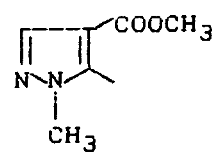 | $OCH_3$ | $CF_3$ | |
| 131 | CH | 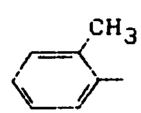 | $CH_2CH_2$ | H | 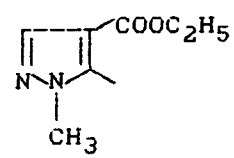 | $OCH_3$ | $CF_3$ | |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 132 | CH | 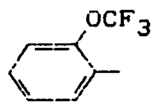 | $CH_2$ | H | 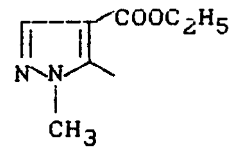 | $OCH_3$ | $CF_3$ | |
| 133 | CH | 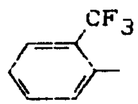 | $CH_2CH_2$ | H | 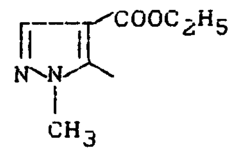 | $OCH_3$ | $CF_3$ | |
| 134 | CH | 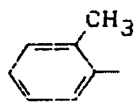 | $CH_3$ | H | 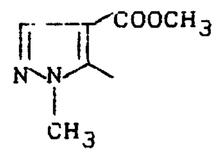 | $OCH_3$ | $CF_3$ | |
| 135 | CH | 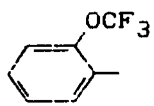 | $CH_3$ | H | 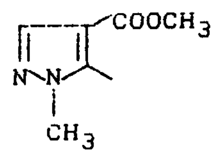 | $OCH_3$ | $CF_3$ | |
| 136 | CH | 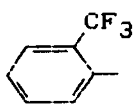 | $CH_2CH_2$ | H | 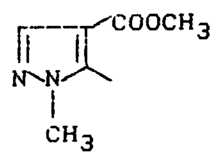 | $OCH_3$ | $CF_3$ | |

<u>Tabelle 1</u> - Fortsetzung

| Bsp.-Nr. | A | Ar | E | M | $R^1$ | $R^2$ | $R^3$ | Schmelzpunkt (°C) |
|---|---|---|---|---|---|---|---|---|
| 137 | CH | 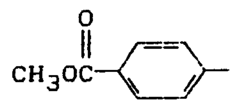 | $CH_2$ | H | 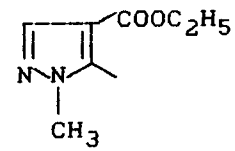 | $OCH_3$ | $CF_3$ | |
| 138 | CH | 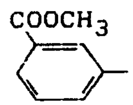 | $CH_2$ | H | 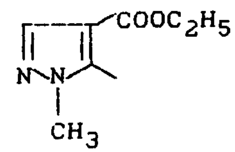 | $OCH_3$ | $CF_3$ | |
| 139 | CH | 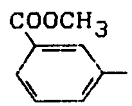 | $CH_2$ | H | 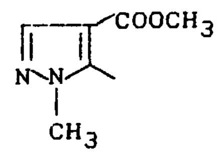 | $OCH_3$ | $CF_3$ | |
| 140 | CH | 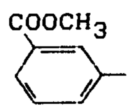 | $CH_2$ | H | 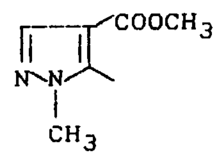 | $OCH_3$ | $CF_3$ | |
| 141 | CH | 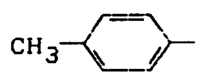 | $CH_2$ | H | 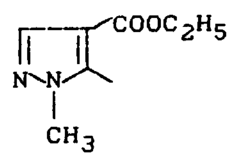 | $OCH_3$ | $CF_3$ | |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 142 | CH | 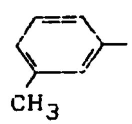 | $CH_2$ | H | 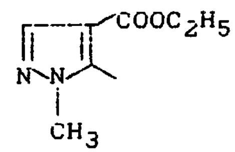 | $OCH_3$ | $CF_3$ | |
| 143 | CH | 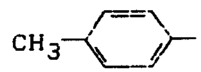 | $CH_2$ | H | 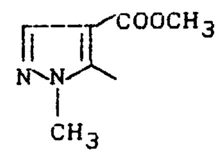 | $OCH_3$ | $CF_3$ | |
| 144 | CH | 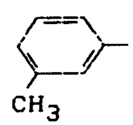 | $CH_2$ | H | 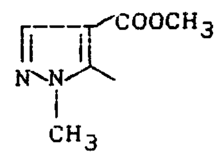 | $OCH_3$ | $CF_3$ | |
| 145 | CH | 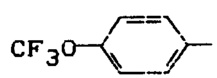 | $CH_2$ | H | 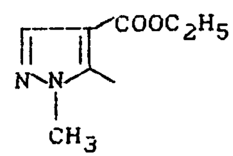 | $OCH_3$ | $CF_3$ | |
| 146 | CH | 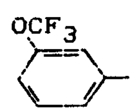 | $CH_2$ | H | 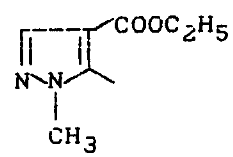 | $OCH_3$ | $CF_3$ | |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 147 | CH | 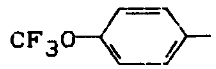 | $CH_2$ | H | 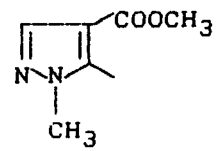 | $OCH_3$ | $CF_3$ | |
| 148 | CH | 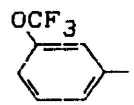 | $CH_2$ | H | 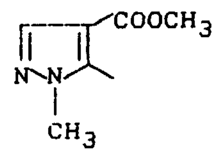 | $OCH_3$ | $CF_3$ | |
| 149 | CH | 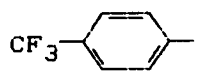 | $CH_2$ | H | 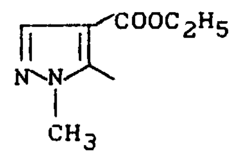 | $OCH_3$ | $CF_3$ | |
| 150 | CH | 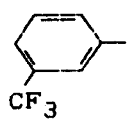 | $CH_2$ | H | 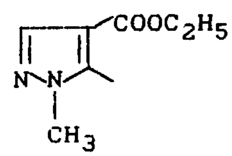 | $OCH_3$ | $CF_3$ | |
| 151 | CH | 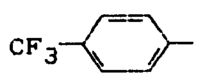 | $CH_2$ | H | 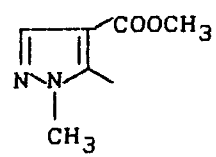 | $OCH_3$ | $CF_3$ | |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 152 | N | 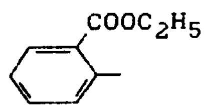 | $CH_2$ | H | 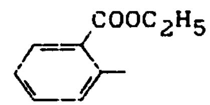 | $CH_3$ | $OCH_3$ | |
| 153 | N | 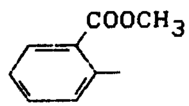 | $CH_2CH_2$ | H | 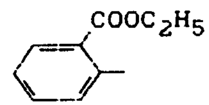 | $CH_3$ | $OCH_3$ | |
| 154 | N | 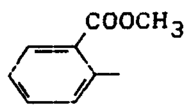 | $CH_2$ | H | 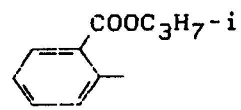 | $CH_3$ | $OCH_3$ | |
| 155 | N | 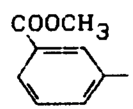 | $CH_2$ | H | 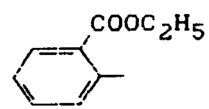 | $CH_3$ | $OCH_3$ | |
| 156 | N | 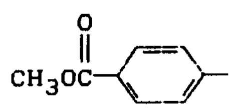 | $CH_2$ | H | 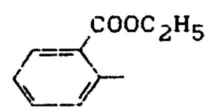 | $CH_3$ | $OCH_3$ | |
| 157 | N | 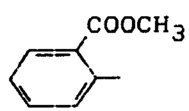 | $CH_2$ | H | 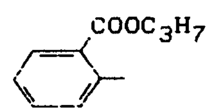 | $CH_3$ | $OCH_3$ | |

<u>Tabelle 1</u> - Fortsetzung

| Bsp.-Nr. | A | Ar | E | M | $R^1$ | $R^2$ | $R^3$ | Schmelzpunkt (°C) |
|---|---|---|---|---|---|---|---|---|
| 158 | N | 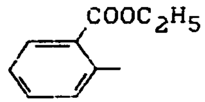 | $CH_2$ | H | 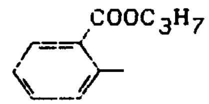 | $CH_3$ | $OCH_3$ | |
| 159 | CH | 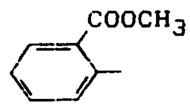 | $CH_2$ | H | 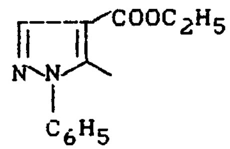 | $OCH_3$ | $OCH_3$ | 135 |
| 160 | CH | 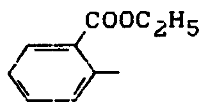 | $CH_2$ | H | 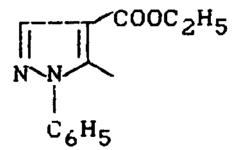 | $OCH_3$ | $OCH_3$ | 118 |
| 161 | CH | 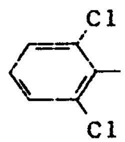 | $CH_2$ | H | 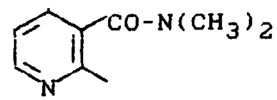 | $OCH_3$ | $OCH_3$ | 120 |
| 162 | CH | 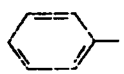 | $CH_2$ | H | 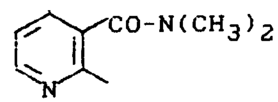 | $OCH_3$ | $OCH_3$ | 151 |
| 163 | CH | 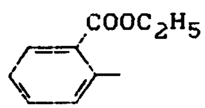 | $CH_2$ | H | 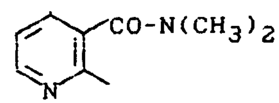 | $OCH_3$ | $OCH_3$ | 142 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 164 | CH | 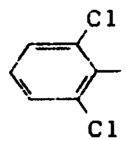 | $CH_2$ | H | 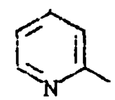 | $OCH_3$ | $OCH_3$ | 153 |
| 165 | CH | 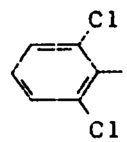 | $CH_2$ | H | 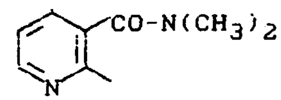 | $CH_3$ | $OCH_3$ | 194 |
| 166 | CH | 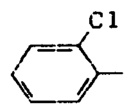 | $CH_2$ | H | 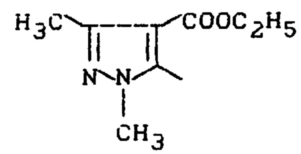 | $CH_3$ | $OCH_3$ | 88 |
| 167 | CH | 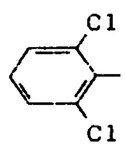 | $CH_2$ | H | 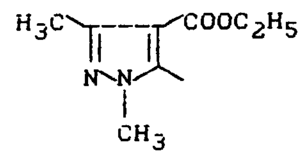 | $CH_3$ | $OCH_3$ | 106 |
| 168 | CH | 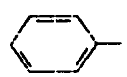 | $CH_2CH_2$ | H | 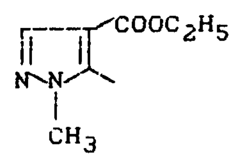 | $CH_3$ | $OCH_3$ | 148 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 169 | CH | 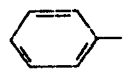 | $CH_2CH_2$ | H | 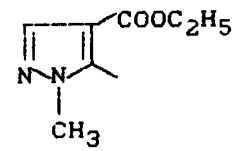 | $OCH_3$ | $OCH_3$ | 143 |
| 170 | CH | 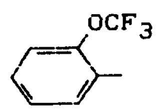 | $CH_2$ | H | 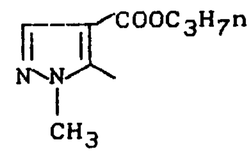 | $OCH_3$ | $OCH_3$ | 121 |
| 171 | CH | 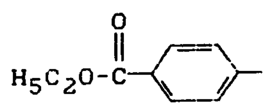 | $CH_2$ | H | 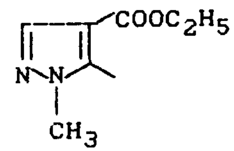 | $CH_3$ | $OCH_3$ | 148 |
| 172 | CH | 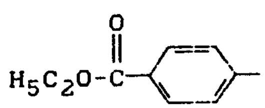 | $CH_2$ | H | 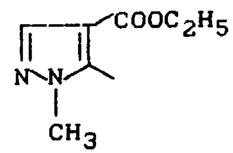 | $OCH_3$ | $OCH_3$ | 177 |
| 173 | CH | 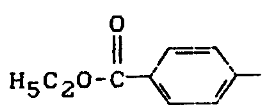 | $CH_2$ | H | 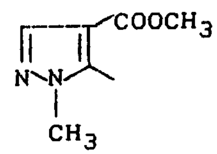 | $CH_3$ | $OCH_3$ | 178 |

**Tabelle 1** - Fortsetzung

| Bsp.-Nr. | A | Ar | E | M | $R^1$ | $R^2$ | $R^3$ | Schmelzpunkt (°C) |
|---|---|---|---|---|---|---|---|---|
| 174 | CH | 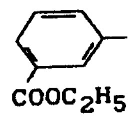 | $CH_2$ | H | 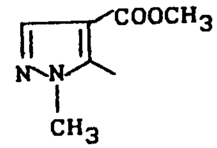 | $OCH_3$ | $OCH_3$ | 160 |
| 175 | CH | 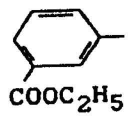 | $CH_2$ | H | 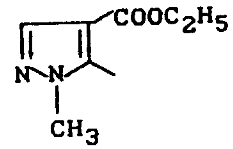 | $CH_3$ | $OCH_3$ | 138 |
| 176 | CH | 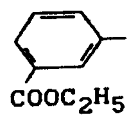 | $CH_2$ | H | 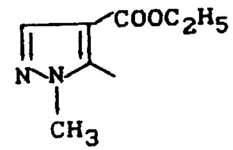 | $OCH_3$ | $OCH_3$ | 157 |
| 177 | CH | 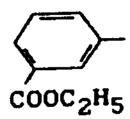 | $CH_2$ | H | 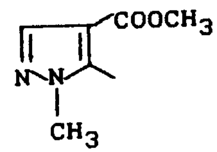 | $OCH_3$ | $OCH_3$ | 159 |
| 178 | CH | 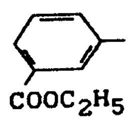 | $CH_2$ | H | 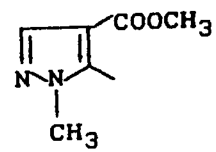 | $CH_3$ | $OCH_3$ | 135 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 179 | CH | 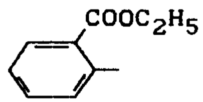 | $CH_2$ | H | 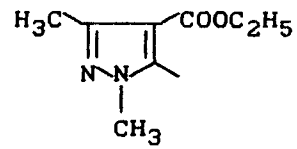 | $OCH_3$ | $OCH_3$ | 140 |
| 180 | CH | 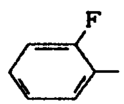 | $CH_2$ | H | 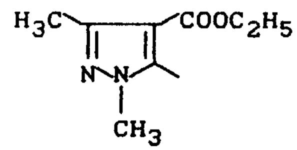 | $CH_3$ | $OCH_3$ | 168 |
| 181 | CH | 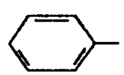 | $CH_2$ | H | 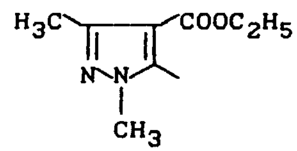 | $CH_3$ | $OCH_3$ | 158 |
| 182 | CH | 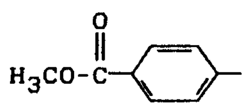 | $CH_2$ | H | 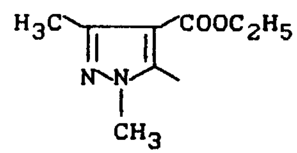 | $CH_3$ | $OCH_3$ | 188 |
| 183 | CH | 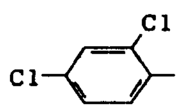 | $CH_2$ | H | 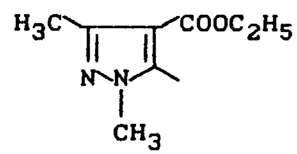 | $CH_3$ | $OCH_3$ | 161 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 184 | CH | 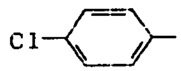 | $CH_2$ | H | 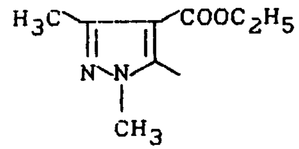 | $CH_3$ | $OCH_3$ | 135 |
| 185 | CH | 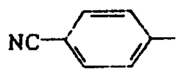 | $CH_2$ | H | 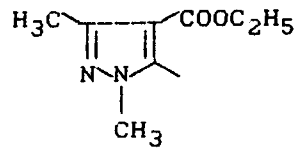 | $CH_3$ | $OCH_3$ | 212 |
| 186 | CH | 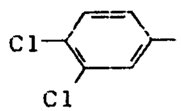 | $CH_2$ | H | 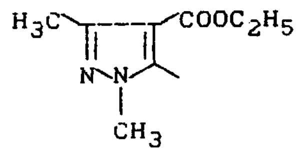 | $CH_3$ | $OCH_3$ | 146 |
| 187 | CH | 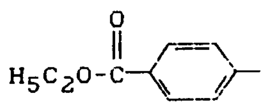 | $CH_2$ | H | 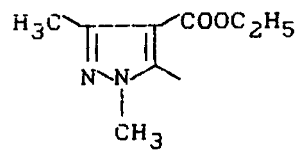 | $CH_3$ | $OCH_3$ | 174 |
| 188 | CH | 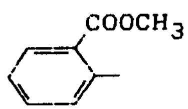 | $CH_2$ | H | 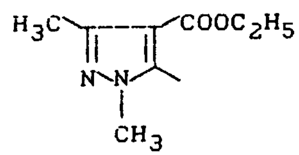 | $CH_3$ | $OCH_3$ | 149 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 189 | CH | 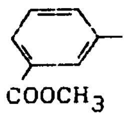 | $CH_2$ | H | 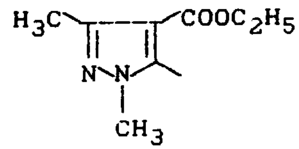 | $CH_3$ | $OCH_3$ | 133 |
| 190 | CH | 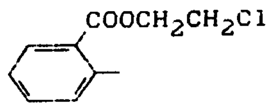 | $CH_2$ | H | 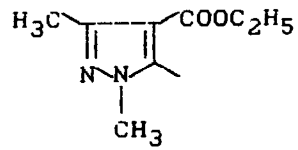 | $CH_3$ | $OCH_3$ | 142 |
| 191 | CH | 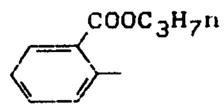 | $CH_2$ | H | 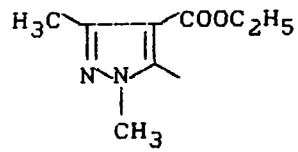 | $CH_3$ | $OCH_3$ | 139 |
| 192 | CH | 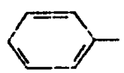 | $CH_2CH_2$ | H | 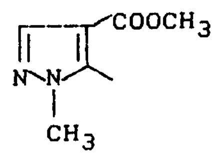 | $OCH_3$ | $OCH_3$ | 150 |
| 193 | CH | 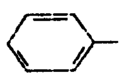 | $CH_2CH_2$ | H | 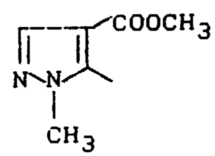 | $CH_3$ | $OCH_3$ | 102 |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 194 | N | 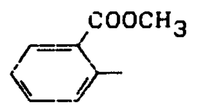 | $CH_2$ | H | 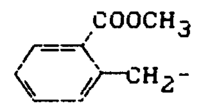 | $OCH_3$ | $OCH_3$ | |
| 195 | N | 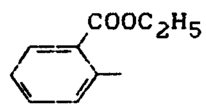 | $CH_2$ | H | 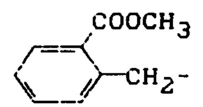 | $OCH_3$ | $OCH_3$ | |
| 196 | N | 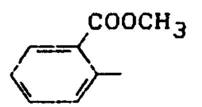 | $CH_2$ | H | 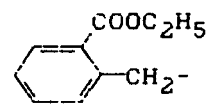 | $OCH_3$ | $OCH_3$ | |
| 197 | N | 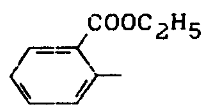 | $CH_2$ | H | 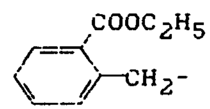 | $OCH_3$ | $OCH_3$ | |
| 198 | N | 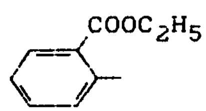 | $CH_2$ | H | 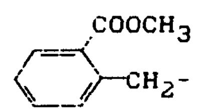 | $CH_3$ | $OCH_3$ | |
| 199 | N | 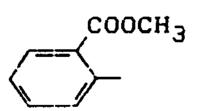 | $CH_2$ | H | 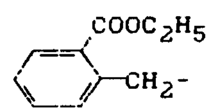 | $CH_3$ | $OCH_3$ | |

Tabelle 1 - Fortsetzung

| Bsp.-Nr. | A | Ar | E | M | $R^1$ | $R^2$ | $R^3$ | Schmelzpunkt (°C) |
|---|---|---|---|---|---|---|---|---|
| 200 | N | 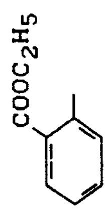 | $CH_2$ | H | 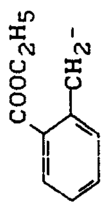 | $CH_3$ | $OCH_3$ | |
| 201 | CH | 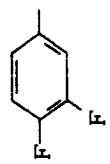 | $CH_2$ | H | 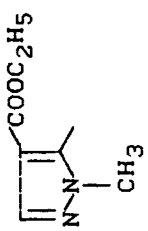 | $OCH_3$ | $OCH_3$ | 147 |
| 202 | CH | 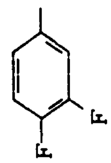 | $CH_2$ | H | 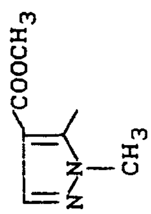 | $OCH_3$ | $OCH_3$ | |
| 203 | CH | 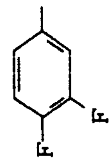 | $CH_2$ | H | 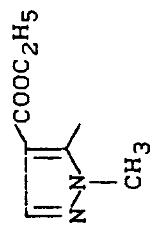 | $CH_3$ | $OCH_3$ | 171 |

Die in Tabelle 1 als Beispiel Nr. 168 aufgeführte Verbindung kann beispielsweise wie folgt hergestellt werden:

Beispiel 168

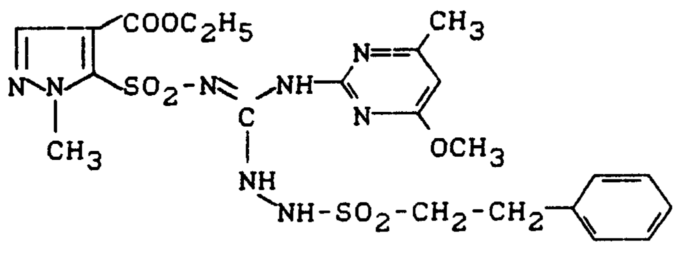

6,2 g (15 mMol) N'-(4-Methoxy-6-methyl-pyrimidin-2-yl)-N''-amino-N'''-(1-methyl-4-ethoxycarbonyl-pyrazol-5-yl-sulfonyl)-guanidin werden in 100 ml Methylenchlorid gelöst und bei -5°C zunächst mit 1,2 ml Pyridin und dann mit 3,0 g (15 mMol) 2-Phenyl-ethansulfonsäurechlorid versetzt. Das Reaktionsgemisch wird 2 Stunden bei -5°C und dann 20 Stunden bei 20°C gerührt. Nach Einengen wird der Rückstand durch Digerieren mit Methanol zur Kristallisation gebracht und das Produkt durch Absaugen isoliert.

Man erhält 3,8 g (44% der Theorie) N'-(4-Methoxy-6-methyl-pyrimidin-2-yl)-N''-(2-phenyl-ethyl-sulfonylamino)-N'''-(1-methyl-4-ethoxycarbonyl-pyrazol-5-yl-sulfonyl)-guanidin vom Schmelzpunkt 148°C.

Anwendungsbeispiele:

In den folgenden Anwendungsbeispielen wird die nachstehend aufgeführte Verbindung als Vergleichssubstanz herangezogen:

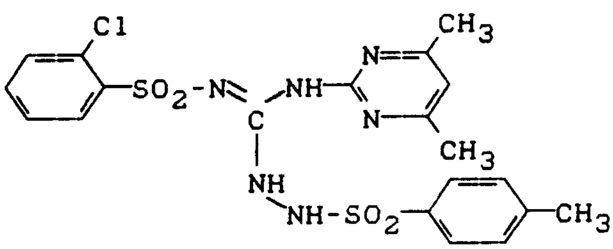

(A)

N'-(4,6-Dimethyl-pyrimidin-2-yl)-N''-(4-methyl-phenylsulfonylamino)-N'''-(2-chlor-phenylsulfonyl)-guanidin (bekannt aus EP-A 121 082).

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem

Stand der Technik zeigen in diesem Test z.B. die Verbindungen gemäß folgender Herstellungsbeispiele: 1, 2, 5, 6, 8, 11 und 17.

**Patentansprüche**

**1.** Substituierte Aralkylsulfonylaminoguanidinoazine der allgemeinen Formel (I),

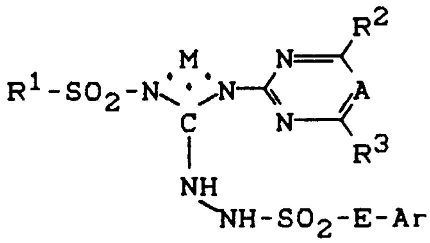

(I)

in welcher

| | |
|---|---|
| A | für Stickstoff oder eine CH-Gruppierung steht, |
| Ar | für gegebenenfalls substituiertes Aryl steht, |
| E | für Alkandiyl (Alkylen) steht, |
| M | für Wasserstoff, ein Metalläquivalent, ein Ammonium-, Alkylammonium-, Dialkylammonium- oder Trialkylammonium-Äquivalent steht, |
| $R^1$ | für jeweils gegebenenfalls substituiertes Aryl, Aralkyl oder Heteroaryl steht, sowie |
| $R^2$ und $R^3$ | gleich oder verschieden sind und für Wasserstoff, Halogen, Alkyl, Halogenalkyl, Alkoxyalkyl, Alkoxy, Halogenalkoxy, Alkoxyalkoxy, Alkylthio, Amino, Alkylamino oder Dialkylamino stehen. |

**2.** Substituierte Aralkylsulfonylaminoguanidinoazine der Formel (I) gemäß Anspruch 1, dadurch gekennzeichnet, daß darin

| | |
|---|---|
| A | für Stickstoff oder eine CH-Gruppierung steht, |
| Ar | für jeweils gegebenenfalls einfach oder zweifach substituiertes Phenyl oder Naphthyl steht, wobei die Substituenten vorzugsweise ausgewählt sind aus der Reihe Fluor, Chlor, Brom, Cyano, Nitro, Carboxy, Carbamoyl, jeweils gegebenenfalls durch Fluor und/oder Chlor einfach bis dreifach substituiertes $C_1$-$C_4$-Alkyl oder $C_1$-$C_4$-Alkoxy, jeweils gegebenenfalls durch Fluor, Chlor, Methoxy oder Ethoxy substituiertes $C_1$-$C_4$-Alkoxy-carbonyl oder $C_1$-$C_4$-Alkylamino-carbonyl, |
| E | für $C_1$-$C_3$-Alkandiyl steht, |
| M | für Wasserstoff oder ein Natrium-, Kalium- oder Calciumäquivalent, ein Ammonium-, ein $C_1$-$C_6$-Alkylammonium-, ein Di-($C_1$-$C_4$-alkyl)-ammonium- oder ein Tri-($C_1$-$C_4$-alkyl)-ammonium-äquivalent steht, |
| $R^1$ | für den Rest |

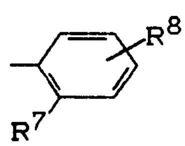

steht, worin

| | |
|---|---|
| $R^7$ und $R^8$ | gleich oder verschieden sind und für Wasserstoff, Fluor, Chlor, Brom, Iod, Cyano, Nitro, $C_1$-$C_6$-Alkyl (welches gegebenenfalls durch Fluor, Chlor, Brom, Cyano, Carboxy, $C_1$-$C_4$-Alkoxycarbonyl, $C_1$-$C_4$-Alkylamino-carbonyl, Di-($C_1$-$C_4$-alkyl)-aminocarbonyl, Hydroxy, $C_1$-$C_4$-Alkoxy, Formyloxy, $C_1$-$C_4$-Alkyl-carbonyloxy, $C_1$-$C_4$-Alkoxy-carbonyloxy, $C_1$-$C_4$-Alkylamino-carbonyloxy, $C_1$-$C_4$-Alkylthio, $C_1$-$C_4$-Alkylsulfinyl, $C_1$-$C_4$-Alkylsulfonyl, Di-($C_1$-$C_4$-alkyl)aminosulfonyl, $C_3$-$C_6$-Cycloalkyl oder Phenyl substituiert ist), für $C_2$-$C_6$-Alkenyl (welches gegebenenfalls durch Fluor, Chlor, Brom, Cyano, $C_1$-$C_4$-Alkoxy-carbonyl, Carboxy oder Phenyl substituiert ist), für $C_2$-$C_6$-Alkinyl |

(welches gegebenenfalls durch Fluor, Chlor, Brom, Cyano, $C_1$-$C_4$-Alkoxy-carbonyl, Carboxy oder Phenyl substituiert ist), für $C_1$-$C_4$-Alkoxy (welches gegebenenfalls durch Fluor, Chlor, Brom, Cyano, Carboxy, $C_1$-$C_4$-Alkoxy-carbonyl, $C_1$-$C_4$-Alkoxy, $C_1$-$C_4$-Alkylthio, $C_1$-$C_4$-Alkylsulfinyl oder $C_1$-$C_4$-Alkylsulfonyl substituiert ist), für $C_1$-$C_4$-Alkylthio (welches gegebenenfalls durch Fluor, Chlor, Brom, Cyano, Carboxy, $C_1$-$C_4$-Alkoxy-carbonyl, $C_1$-$C_4$-Alkylthio, $C_1$-$C_4$-Alkylsulfinyl oder $C_1$-$C_4$-Alkylsulfonyl substituiert ist), für $C_3$-$C_6$-Alkenyloxy (welches gegebenenfalls durch Fluor, Chlor, Brom, Cyano oder $C_1$-$C_4$-Alkoxycarbonyl substituiert ist), für $C_2$-$C_6$-Alkenylthio (welches gegebenenfalls durch Fluor, Chlor, Brom, Cyano, Nitro, $C_1$-$C_3$-Alkylthio oder $C_1$-$C_4$-Alkoxycarbonyl substituiert ist), $C_3$-$C_6$-Alkinyloxy, $C_3$-$C_6$-Alkinylthio oder für den Rest $-S(O)_p-R^9$ stehen, wobei

p für die Zahlen 1 oder 2 steht und

$R^9$ für $C_1$-$C_4$-Alkyl (welches gegebenenfalls durch Fluor, Chlor, Brom, Cyano oder $C_1$-$C_4$-Alkoxy-carbonyl substituiert ist), $C_3$-$C_6$-Alkenyl, $C_3$-$C_6$-Alkinyl, $C_1$-$C_4$-Alkoxy, $C_1$-$C_4$-Alkoxy-$C_1$-$C_4$-alkylamino, $C_1$-$C_4$-Alkylamino, Di-($C_1$-$C_4$-alkyl)-amino oder für den Rest $-NHOR^{10}$ steht, wobei

$R^{10}$ für $C_1$-$C_6$-Alkyl (welches gegebenenfalls durch Fluor, Chlor, Cyano, $C_1$-$C_4$-Alkoxy, $C_1$-$C_4$-Alkylthio, $C_1$-$C_4$-Alkylsulfinyl, $C_1$-$C_4$-Alkylsulfonyl, $C_1$-$C_4$-Alkyl-carbonyl, $C_1$-$C_4$-Alkoxy-carbonyl, $C_1$-$C_4$-Alkylamino-carbonyl oder Di-($C_1$-$C_4$-alkyl)-amino-carbonyl substituiert ist), für $C_3$-$C_6$-Alkenyl (welches gegebenenfalls durch Fluor, Chlor oder Brom substituiert ist), $C_3$-$C_6$-Alkinyl, $C_3$-$C_6$-Cycloalkyl, $C_3$-$C_6$-Cycloalkyl-$C_1$-$C_2$-alkyl, Phenyl-$C_1$-$C_2$-alkyl (welches gegebenenfalls durch Fluor, Chlor, Nitro, Cyano, $C_1$-$C_4$-Alkyl, $C_1$-$C_4$-Alkoxy oder $C_1$-$C_4$-Alkoxy-carbonyl substituiert ist), für Benzhydryl oder für Phenyl (welches gegebenenfalls durch Fluor, Chlor, Nitro, Cyano, $C_1$-$C_4$-Alkyl, Trifluormethyl, $C_1$-$C_4$-Alkoxy, $C_1$-$C_2$-Fluoralkoxy, $C_1$-$C_4$-Alkylthio, Trifluormethylthio oder $C_1$-$C_4$-Alkoxy-carbonyl substituiert ist) steht,

$R^7$ und $R^8$ weiterhin für Phenyl oder Phenoxy, für $C_1$-$C_4$-Alkylcarbonylamino, $C_1$-$C_4$-Alkoxycarbonylamino, $C_1$-$C_4$-Alkylamino-carbonylamino, Di-($C_1$-$C_4$-alkyl)-amino-carbonylamino, oder für den Rest $-CO-R^{11}$ stehen, wobei

$R^{11}$ für $C_1$-$C_6$-Alkyl, $C_1$-$C_6$-Alkoxy, $C_3$-$C_6$-Cycloalkoxy, $C_3$-$C_6$-Alkenyloxy, $C_1$-$C_4$ Alkylthio, Amino, $C_1$-$C_4$-Alkylamino, $C_1$-$C_4$-Alkoxyamino, $C_1$-$C_4$-Alkoxy-$C_1$-$C_4$-alkyl-amino oder Di-($C_1$-$C_4$-alkyl)-amino steht (welche gegebenenfalls durch Fluor und/oder Chlor substituiert sind),

$R^7$ und $R^8$ weiterhin für $C_1$-$C_4$-Alkylsulfonyloxy, Di-($C_1$-$C_4$-alkyl)-aminosulfonylamino oder für den Rest $-CH-N-R^{12}$ stehen, wobei

$R^{12}$ für gegebenenfalls durch Fluor, Chlor, Cyano, Carboxy, $C_1$-$C_4$-Alkoxy, $C_1$-$C_4$-Alkylthio, $C_1$-$C_4$-Alkylsulfinyl oder $C_1$-$C_4$-Alkylsulfonyl substituiertes $C_1$-$C_6$-Alkyl, für gegebenenfalls durch Fluor oder Chlor substituiertes Benzyl, für gegebenenfalls durch Fluor oder Chlor substituiertes $C_3$-$C_6$-Alkenyl oder $C_3$-$C_6$-Alkinyl, für gegebenenfalls durch Fluor, Chlor, Brom, $C_1$-$C_4$-Alkyl, $C_1$-$C_4$-Alkoxy, Trifluormethyl, Trifluormethoxy oder Trifluormethylthio substituiertes Phenyl, für gegebenenfalls durch Fluor und/oder Chlor substituiertes $C_1$-$C_6$-Alkoxy, $C_3$-$C_6$-Alkenoxy, $C_3$-$C_6$-Alkinoxy oder Benzyloxy, für Amino, $C_1$-$C_4$-Alkylamino, Di-($C_1$-$C_4$-alkyl)-amino, Phenylamino, $C_1$-$C_4$-Alkylcarbonyl-amino, $C_1$-$C_4$-Alkoxy-carbonylamino, $C_1$-$C_4$-Alkyl-sulfonylamino oder für gegebenenfalls durch Fluor, Chlor, Brom oder Methyl substituiertes Phenylsulfonylamino steht,

weiter

$R^1$ für den Rest

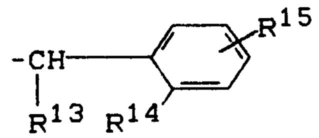

steht, worin

R[13] für Wasserstoff oder $C_1$-$C_4$-Alkyl steht,

$R^{14}$ und $R^{15}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_1$-$C_4$-Alkoxy (welches gegebenenfalls durch Fluor und/oder Chlor substituiert ist), Carboxy, $C_1$-$C_4$-Alkoxy-carbonyl, $C_1$-$C_4$-Alkylsulfonyl oder Di-($C_1$-$C_4$-alkyl)-aminosulfonyl stehen;

weiter

$R^1$ für den Rest

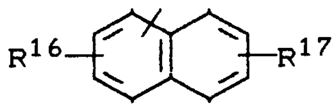

steht, worin

$R^{16}$ und $R^{17}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist) oder $C_1$-$C_4$-Alkoxy (welches gegebenenfalls durch Fluor und/oder Chlor substituiert ist), stehen;

weiter

$R^1$ für den Rest

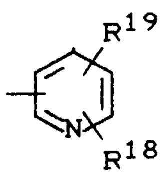

steht, worin

$R^{18}$ und $R^{19}$ gleich oder verschieden sind und für Wasserstoff, Fluor, Chlor, Brom, Nitro, Cyano, $C_1$-$C_4$-Alkyl (welches gegebenenfalls durch Fluor und/oder Chlor substituiert ist), $C_2$-$C_4$-Alkenyl (welches gegebenenfalls durch Fluor und/oder Chlor substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), sowie für Di-($C_1$-$C_4$-alkyl)-aminosulfonyl, $C_1$-$C_4$-Alkoxy-carbonyl, Dimethylaminocarbonyl oder Dioxolanyl stehen;

weiter

$R^1$ für den Rest

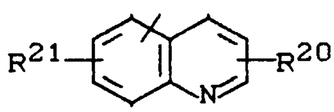

steht, worin

$R^{20}$ und $R^{21}$ gleich oder verschieden sind und für Wasserstoff, Fluor, Chlor, Brom, $C_1$-$C_4$-Alkyl (welches gegebenenfalls durch Fluor und/oder Brom substituiert ist), $C_1$-$C_4$-Alkoxy (welches gegebenenfalls durch Fluor und/oder Chlor substituiert ist), für $C_1$-$C_4$-Alkylthio, $C_1$-$C_4$-Alkylsulfinyl oder $C_1$-$C_4$-Alkylsulfonyl (welche gegebenenfalls durch Fluor und/oder Chlor substituiert sind), oder für Di-($C_1$-$C_4$-alkyl)-aminosulfonyl ste-

hen;

weiter

$R^1$ für den Rest

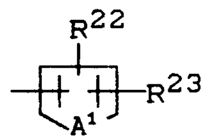

steht, worin

$R^{22}$ und $R^{23}$ gleich oder verschieden sind und für Wasserstoff, Fluor, Chlor, Brom, Cyano, Nitro, $C_1$-$C_4$-Alkyl (welches gegebenenfalls durch Fluor, Chlor, $C_1$-$C_4$-Alkoxy und/oder $C_1$-$C_4$-Halogenalkoxy substituiert ist), $C_1$-$C_4$-Alkoxy (welches gegebenenfalls durch Fluor und/oder Chlor substituiert ist), $C_1$-$C_4$-Alkylthio, $C_1$-$C_4$-Alkylsulfinyl oder $C_1$-$C_4$-Alkylsulfonyl (welches gegebenenfalls durch Fluor und/oder Chlor substituiert ist), Di-($C_1$-$C_4$-alkyl)-amino-sulfonyl oder $C_1$-$C_4$-Alkoxy-carbonyl stehen, und

$A^1$ für Sauerstoff, Schwefel oder die Gruppierung N-$Z^1$ steht, wobei

$Z^1$ für Wasserstoff, $C_1$-$C_4$-Alkyl (welches gegebenenfalls durch Fluor, Chlor, Brom oder Cyano substituiert ist), $C_3$-$C_6$-Cycloalkyl, Benzyl, Phenyl (welches gegebenenfalls durch Fluor, Chlor, Brom oder Nitro substituiert ist), $C_1$-$C_4$-Alkylcarbonyl, $C_1$-$C_4$-Alkoxy-carbonyl oder Di-($C_1$-$C_4$-alkyl)-aminocarbonyl steht;

weiter

$R^1$ für den Rest

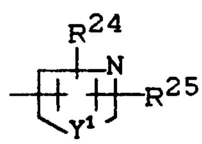

steht, worin

$R^{24}$ und $R^{25}$ gleich oder verschieden sind und für Wasserstoff, $C_1$-$C_4$-Alkyl, Halogen, $C_1$-$C_4$-Alkoxy-carbonyl, $C_1$-$C_4$-Alkoxy oder $C_1$-$C_4$-Halogenalkoxy stehen,

$Y^1$ für Schwefel oder die Gruppierung N-$R^{26}$ steht, wobei

$R^{26}$ für Wasserstoff oder $C_1$-$C_4$-Alkyl steht;

weiter

$R^1$ für den Rest

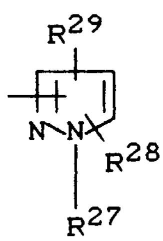

steht, worin

$R^{27}$ für Wasserstoff, $C_1$-$C_4$-Alkyl, Phenyl oder (Iso)Chinolinyl steht,

$R^{28}$ für Wasserstoff, Halogen, Cyano, Nitro, $C_1$-$C_4$-Alkyl (welches gegebenenfalls durch Fluor und/oder Chlor substituiert ist), $C_1$-$C_4$-Alkoxy (welches gegebenenfalls durch

Fluor und/oder Chlor substituiert ist), Dioxolanyl oder $C_1$-$C_4$-Alkoxy-carbonyl steht und

$R^{29}$ für Wasserstoff, Halogen oder $C_1$-$C_4$-Alkyl steht; sowie

$R^2$ und $R^3$ gleich oder verschieden sind und für Wasserstoff, Fluor, Chlor, Brom, Methyl, Ethyl, Trifluormethyl, Methoxymethyl, Methoxy, Ethoxy, Difluormethoxy, Methoxyethoxy, Methylthio, Ethylthio, Amino, Methylamino, Ethylamino oder Dimethylamino stehen.

3. Substituierte Aralkylsulfonylaminoguanidinoazine der Formel (I) gemäß Anspruch 1, dadurch gekennzeichnet, daß darin

A für Stickstoff oder eine CH-Gruppierung steht,

Ar für gegebenenfalls einfach oder zweifach substituiertes Phenyl steht, wobei die Substituenten vorzugsweise ausgewählt sind aus der Reihe Fluor, Chlor, Brom, Carboxy, Methyl, Trifluormethyl, Methoxy, Difluormethoxy, Trifluormethoxy, Methoxycarbonyl, Ethoxycarbonyl und Cyano,

E für $-CH_2-$ oder $-CH_2CH_2-$ steht,

M für Wasserstoff steht,

$R^1$ für den Rest

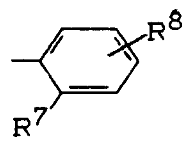

steht, worin

$R^7$ für Fluor, Chlor, Brom, Methyl, Trifluormethyl, Methoxy, Difluormethoxy, Trifluormethoxy, $C_1$-$C_3$-Alkylthio, $C_1$-$C_3$-Alkylsulfinyl, $C_1$-$C_3$-Alkylsulfonyl, Dimethylaminosulfonyl, N-Methoxy-N-methylaminosulfonyl, Phenyl, Phenoxy oder $C_1$-$C_3$-Alkoxy-carbonyl steht und

$R^8$ für Wasserstoff, Fluor oder Chlor steht;

weiter

$R^1$ für den Rest

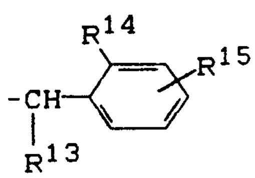

steht, worin

$R^{13}$ für Wasserstoff steht,

$R^{14}$ für Fluor, Chlor, Brom, Methyl, Methoxy, Difluormethoxy, Trifluormethoxy, Ethoxy, Methoxycarbonyl, Ethoxycarbonyl, Methylsulfonyl oder Dimethylaminosulfonyl steht und

$R^{15}$ für Wasserstoff, Fluor oder Chlor steht;

weiter

$R^1$ für den Rest

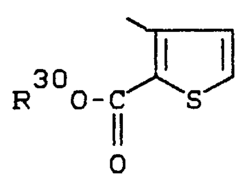

steht, worin

$R^{30}$ für Methyl oder Ethyl steht, oder

$R^1$ für den Rest

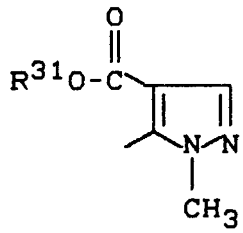

steht, worin

- $R^{31}$ für Methyl oder Ethyl steht,
- $R^2$ für Wasserstoff, Chlor, Methyl, Ethyl, Trifluormethyl, Methoxy oder Ethoxy steht und
- $R^3$ für Methyl, Ethyl, Methoxy oder Ethoxy steht.

**4.** Verfahren zur Herstellung von substituierten Aralkylsulfonylaminoguanidinoazinen der Formel (I) gemäß Anspruch 1, dadurch gekennzeichnet, daß man

(a) Sulfonylverbindungen der allgemeinen Formel (II)

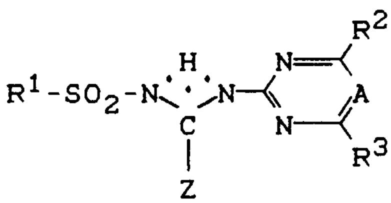

(II)

in welcher

- A, $R^1$, $R^2$ und $R^3$ die oben angegebenen Bedeutungen haben und
- Z für eine der nachstehend angegebenen Abgangsgruppen

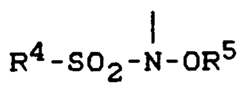

oder -Q-$R^6$ steht,

worin

- $R^4$ die oben für $R^1$ angegebene Bedeutung hat, aber nicht in jedem Einzelfall mit $R^1$ identisch sein muß,
- $R^5$ für Alkyl, Alkenyl oder Aralkyl steht,
- $R^6$ für Alkyl, Aralkyl oder Aryl steht und
- Q für Sauerstoff oder Schwefel steht,

mit Sulfonsäurehydraziden der allgemeinen Formel (III)

$H_2N-NH-SO_2-E-Ar$ (III)

in welcher

- Ar und E die oben angegebene Bedeutung haben,

gegebenenfalls in Gegenwart eines Verdünnungsmittels umsetzt, oder daß man

(b) Aminoguanidinoazine der allgemeinen Formel (IV)

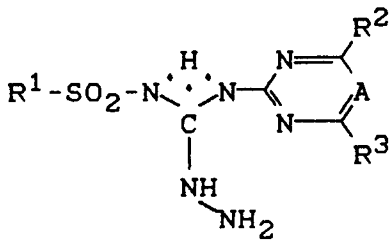

(IV)

in welcher
A, $R^1$, $R^2$ und $R^3$ die oben angegebenen Bedeutungen haben,

mit Sulfonsäurehalogeniden der allgemeinen Formel (V)

$X-SO_2-E-Ar$ (V)

in welcher
Ar und E die oben angegebene Bedeutung haben und
X für Halogen steht,

gegebenenfalls in Gegenwart eines Verdünnungsmittels und gegebenenfalls in Gegenwart eines Säureakzeptors umsetzt,

und gegebenenfalls die so erhaltenen Produkte nach üblichen Methoden in Salze überführt.

**5.** Herbizide Mittel, gekennzeichnet durch einen Gehalt an mindestens einem substituierten Aralkylsulfonylaminoguanidinoazin der Formel (I) gemäß Anspruch 1.

**6.** Verfahren zur Bekämpfung von Unkräutern, dadurch gekennzeichnet, daß man substituierte Aralkylsulfonylaminoguanidinoazine der Formel (I) gemäß Anspruch 1 auf Unkräuter und/oder ihren Lebensraum einwirken läßt.

**7.** Verwendung von substituierten Aralkylsulfonylaminoguanidinoazinen der Formel (I) gemäß Anspruch 1 zur Bekämpfung von Unkräutern.

**8.** Verfahren zur Herstellung von herbiziden Mitteln, dadurch gekennzeichnet, daß man substituierte Aralkylsulfonylaminoguanidinoazine der Formel (I) gemäß Anspruch 1 mit Streckmitteln und/oder oberflächenaktiven Mitteln vermischt.